(19)

**Europäisches Patentamt**
**European Patent Office**
**Office européen des brevets**

(11) **EP 4 579 682 A1**

(12) **EUROPEAN PATENT APPLICATION**

(43) Date of publication:
**02.07.2025 Bulletin 2025/27**

(21) Application number: **23220881.9**

(22) Date of filing: **31.12.2023**

(51) International Patent Classification (IPC):
**G16H 50/20** *(2018.01)*    **G16H 50/30** *(2018.01)*
**G16H 50/50** *(2018.01)*    **G16H 30/40** *(2018.01)*

(52) Cooperative Patent Classification (CPC):
**G16H 50/30; G16H 30/40; G16H 50/20; G16H 50/50**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicants:
• **University of Ioannina**
  **45110 Ioannina (GR)**
• **University of Belgrade**
  **11000 Belgrade (RS)**
• **BioIRC d.o.o. Kragujevac**
  **34000 Kragujevac (RS)**

(72) Inventors:
• **FOTIADIS, Dimitrios**
  **45110 Ioannina (GR)**
• **POTSIKA, Vassiliki**
  **45500 Ioannina (GR)**
• **TSAKANIKAS, Vassilios**
  **30100 Agrinio (GR)**
• **SIOGKAS, Panagiotis**
  **45500 Ioannina (GR)**

• **PLEOURAS, Dimitrios**
  **45221 Ioannina (GR)**
• **KIGKA, Vassiliki**
  **45333 Ioannina (GR)**
• **SAKELLARIOS, Antonios**
  **45332 Ioannina (GR)**
• **IOSIF, Aggeliki**
  **45332 Ioannina (GR)**
• **DIMOS, Sokratis**
  **45445 Ioannina (GR)**
• **MANTZARIS, Michail**
  **45332 Ioannina (GR)**
• **KONCAR, Igor**
  **11000 Belgrade (RS)**
• **FILIPOVIC, Nenad**
  **34000 Kragujevac (RS)**
• **DJUKIC, Tijana**
  **34000 Kragujevac (RS)**
• **SAVELJIC, Igor**
  **34000 Kragujevac (RS)**
• **TOMASEVIC, Smiljana**
  **34000 Kragujevac (RS)**

(74) Representative: **Dodou, Evdokia**
  **Katsimpiri 3**
  **15561 Cholargos, Athens (GR)**

(54) **METHOD AND SYSTEM FOR THE RISK STRATIFICATION OF ASYMPTOMATIC PATIENTS WITH CAROTID STENOSIS**

(57) Computer-implemented method and apparatus for the risk stratification of asymptomatic patients with carotid stenosis. Said method and apparatus are based on a three-level risk stratification model, which integrates non-image medical data and carotid imaging medical data to provide prediction of plaque volume evolution over time, assessment of the risk for plaque rupture or the risk for future cerebrovascular events, among others. These tools aid the clinician in identifying asymptomatic carotid stenosis patients to whom to recommend ultrasound screening, asymptomatic patients with higher risk for stroke to whom to target surgical intervention versus best medication therapy, and/or for providing advice on the optimal time for the next follow-up screening of asymptomatic patients who are not eligible for surgical intervention.

**FIG 4**

**Description**

TECHNICAL FIELD

The present invention provides methods and apparatuses for the improved stratification of asymptomatic patients with carotid artery disease.

BACKGROUND OF THE INVENTION

Carotid artery disease constitutes one of the primary causes of ischaemic cerebrovascular events and accounts for an estimated 150,000 deaths per year from stroke in Europe and 130,000 in the USA, and an even higher burden in morbidity and healthcare costs, due to long term disability. Atherosclerotic plaques in the carotid artery bifurcations cause progressive narrowing of the vessel lumen, which erode or rupture and subsequently cause thromboembolism in the cerebropetal circulation leading to cerebral infarction in 80% of patients while in 20% the cause of stroke is hemodynamic disturbances of cerebral perfusion. The determining factors for carotid artery disease management are currently the degree of stenosis, the presence and recency of symptoms. Patients with 70% stenosis in their carotid artery, either symptomatic or asymptomatic, are considered to be at high risk of cerebrovascular events and are therefore directed to surgical intervention (carotid endarterectomy - CEA or carotid stenting- CAS). In contrast, patients with <70% stenosis are considered at low-intermediate risk when asymptomatic, and unless other confounding factors exist, they are subjected to medical treatment alone. When the patients are symptomatic with recent events, the cut-off of 50% stenosis is used instead. However, this stratification is rather generic, leading to high levels of unnecessary surgical treatment (estimated as high as 95% for asymptomatic patients and costing annually to the European healthcare systems >2 billion €). On the contrary, potential high levels of undertreatment may occur in patients with lower levels of stenosis, who are also at potentially higher risk of cerebrovascular events. Moreover, the criteria for this stratification have been largely based on clinical trials of the 90's and refer to patient populations whose lifestyle and treatment options has dramatically changed since then. Therefore, relative risks for cerebrovascular events of patients with 50% stenosis need revisiting.

Studies of longitudinal carotid artery disease cohorts have observed a major shift in the underlying pathology of symptomatic (causing transient ischaemic attack (TIA) or stroke) and asymptomatic carotid artery plaques. Although vulnerable plaques exhibiting large lipid cores, a thin fibrous cap and a rupture-prone phenotype, are considered to account for the large majority of symptomatic carotid artery disease, a rapidly increasing proportion of acute clinical events originates from phenotypically stable plaques that lack vulnerable plaque characteristics, but have significant proteoglycan and glycosaminoglycan accumulation and collagen levels (Pasterkamp G et al. Nat Rev Cardiol. 2017 Jan;14(1):21-29.). From 2002-2015, vanLammeren GW et al. (Circulation. 2014;129(22):2269-76) have noted a 50% reduction of lipid-rich plaques and intraplaque haemorrhage in symptomatic and asymptomatic carotid lesions. Conversely, an increasing rate of non-ruptured lesions has been observed that are likely to "erode" and cause thromboembolic complications, through different mechanisms than previously suggested. Notably, these plaques are twice more frequent in women than in men, indicating an important gender dimension. During these years, it has become apparent that from all plaques with the vulnerable phenotype, only a very small proportion of them (estimated at <5%) rupture, whereas the remaining ones do not cause clinical events (Stone GW et al. N Engl J Med. 2011 Jan 20; 364(3):226-35). Similarly, it has been shown that from all vulnerable plaques that rupture, only a fraction of them causes clinical manifestations, while the rest remain clinically silent (Burke AP et al. Circulation. 2001;103(7):934-40). Moreover, it has been demonstrated that vulnerable plaques can lose their vulnerable characteristics, while stable plaques can become vulnerable over time, depending on exogenous factors, such as medical treatment and living behaviour (Mann J & Davies MJ. Heart. 1999 Sep;82(3):265-8)

These new insights into the types of lesions that cause symptoms and time-dependent changes in therapy, lifestyle and underlying pathology of carotid artery stenosis leading to TIA or stroke cannot be ignored. They raise a major unmet need to stratify patients for carotid surgery and medical treatment in the current era, where old rules do not apply and where medical needs are not the same as in the past. This is vital, especially when taking into account the magnitude of morbidity, mortality and socioeconomic costs of this highly prevalent and devastating disease.

SUMMARY OF THE INVENTION

The invention described herein provides a method for multi-level computational method for the risk stratification of asymptomatic patients with carotid artery disease. Specifically, said method comprises the following steps:

a) storing, using a database, medical data including non-image medical data and carotid imaging medical data for a plurality of patients, wherein said non-image medical data comprise demographics, clinical characteristics, data from blood examinations and medication intake;

b) inputting one or more non-image medical data values into a plurality of algorithms to produce a first set of outputs;

c) inputting one or more carotid imaging data values into a plurality of algorithms comprising at least a plaque evolution prediction model for predicting plaque progression, stabilization, or regression; and/or a plaque rupture prediction model utilizing plaque burden metrics such as volume, cross-sectional area and/or thickness of the plaque components, plaque composition metrics and/or biomechanical features; to produce a second set of outputs that provide plaque evolution and risk prediction for plaque rupture; and

d) combining the first set of outputs with the second set of outputs to provide an overall risk estimation for plaque progression, plaque rupture, cerebral and cardiovascular disease outcomes for said asymptomatic patients.

An advantage of the method disclosed herein is that it is highly adapted to the particular characteristics and medical history of each patient, thus providing a personalized risk assessment aid.

An additional advantage of said method is that it provides a stratification tool that has a higher predictive accuracy and comprehensiveness compared to prior art solutions.

Other aspects and benefits of the present invention will become apparent from the detailed description to follow.

BRIEF DESCRIPTION OF THE DRAWINGS

The present invention will now be described with reference to certain embodiments thereof which are illustrated in the accompanying drawings. It should be noted that the accompanying drawings illustrate preferred embodiments of the invention, therefore should not be considered as limiting the scope of the invention.

Fig. 1 is a schematic presentation of the current guidelines for Carotid Artery Disease treatment and gaps where the present invention can contribute to improve standard of care.

Fig. 2 depicts the Current guidelines for Carotid Artery Disease treatment and the invention's contribution to improved Standard of Care.

Fig. 3 is a schematic presentation of the information flow of the Risk Stratification Tool.

Fig. 4 is a schematic presentation of the Risk Stratification tool overall architecture.

Fig. 5 is a flow diagram of the methodology for the 3D reconstruction of the carotid arterial tree.

Fig. 6 is a schematic diagram of the Validation metrics.

Fig. 7 presents the Dice Coefficient.

Fig. 8 shows the results from the study validating the arterial lumen geometry reconstruction using Magnetic Resonance imaging (MRI). (A) Linear Regression analysis of the lumen area for automatic and manual segmentation, (B) Linear Regression analysis of the lumen perimeter for automatic and manual segmentation, (C) Bland Altman Analysis (areas), (D) Bland Altman Analysis (perimeters), (E) Hausdorff Distance, (F) Dice coefficient analysis on lumen area.

Fig. 9 shows the results from the study validating the arterial outer wall geometry reconstruction using MRI. (A) Linear Regression analysis of the outer wall area for automatic and manual segmentation, (B) Linear Regression analysis of the outer wall perimeter for automatic and manual segmentation, (C) Bland Altman Analysis (areas), (D) Bland Altman Analysis (perimeters), Hausdorff Distance, (F) Dice coefficient analysis on outer wall area.

Fig. 10 is a flow diagram showing the overall approach for plaque characterization.

Fig. 11 is a flow diagram of the methodology for plaque characterization using MRI modalities.

Fig. 12 shows the results from the studies validating the methodology for plaque characterization using MRI modalities. (A) Linear Regression analysis of the calcification areas for automatic and manual segmentation and (B) corresponding Bland Altman Analysis (areas), (C) Linear Regression analysis of the fibrosis areas for automatic and manual segmentation and (D) corresponding Bland Altman Analysis (areas), (E) Linear Regression analysis of the lipid core areas for automatic and manual segmentation and (F) corresponding Bland Altman Analysis (areas).

Fig. 13 presents 3D arterial models from sample case studies, where the lumen, the outer wall and the plaque components are presented.

Fig. 14 is a flow diagram of the developed methodology for the reconstruction of carotid bifurcations using US imaging.

Fig. 15 shows (A to C) carotid ultrasound images: (A) original images, (B) lumen masks, (C) wall masks. (D, E) illustrate the evolution of the proposed 3D reconstruction methodology: (D) generalized model proposed in prior art and (E) new improved approach used for creation of patient-specific 3D model.

Fig. 16 shows the reconstructed geometry for two representative patients (A, C and B, D). In (C, D) the reconstructed geometry is shown with separated plaque components. 1: Whole reconstructed mesh with separated plaque region; 2: longitudinal cross-section; 3: transversal cross-section.

Fig. 17 shows (A) a flow diagram for 3D reconstruction of carotid arteries using CTA imaging modality, (B) the 3D reconstruction of a carotid artery bifurcation, (C) the 2D CTA slice without segmentation and (D) the 2D segmentation of the inner wall and outer wall.

Fig. 18 depicts in (A, B) the blood flow validation process. (C, D) depict the regression plot (C) and the Bland-Altman plot of the analysis (D).

Fig. 19 is a flow diagram of the information flow of the developed plaque growth model.

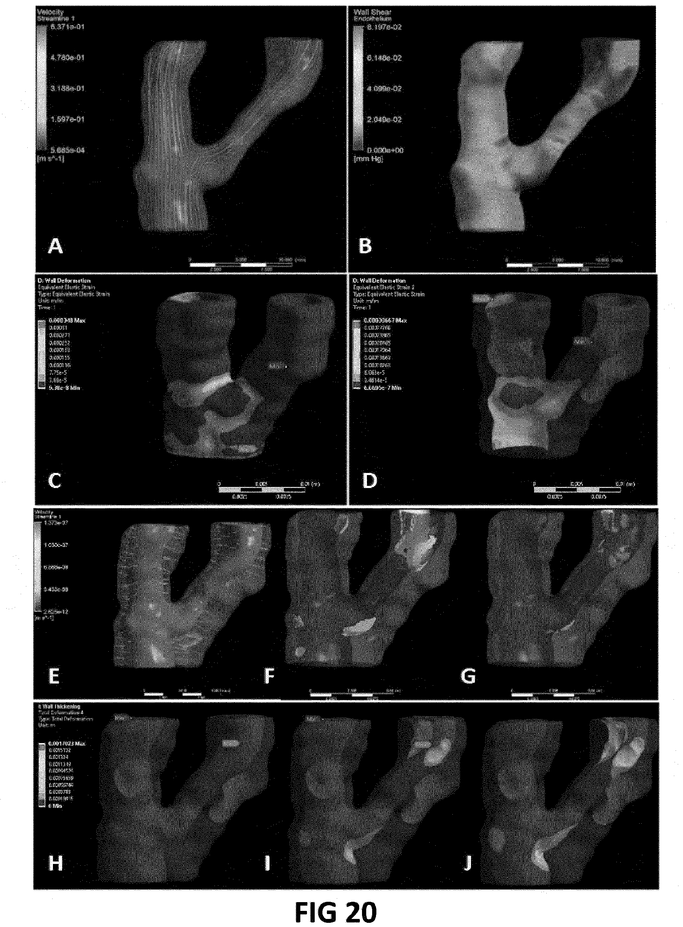

Fig. 20 depicts case examples (A, B) of the streamlines of a 3D reconstructed carotid bifurcation, which are colored according to the magnitude of velocity (A) and the endothelial shear stress distribution (B); the endothelial strain distribution (C) and the arterial wall strain distribution (D); the plasma flow streamlines within the porous arterial wall (E); the predicted lipid (F) and fibrous (G) plaque regions of a 3D reconstructed carotid bifurcation, and (H-J) the progression of lumen stenosis & wall thickening over 1 year of a high-risk patient's 3D reconstructed carotid bifurcation, where (H) is the initial arterial geometry, (I) is 0.5 year later and (J) is 1 year later.

Fig. 21 shows the simulations of atherosclerotic progression using the agent-based model

Fig. 22 shows (A) plaque structural stress 2D analysis for the first frame of plaque burden of >40% for patient #12 and (B) total deformation analysis for the first frame of plaque burden of >40% for the same patient.

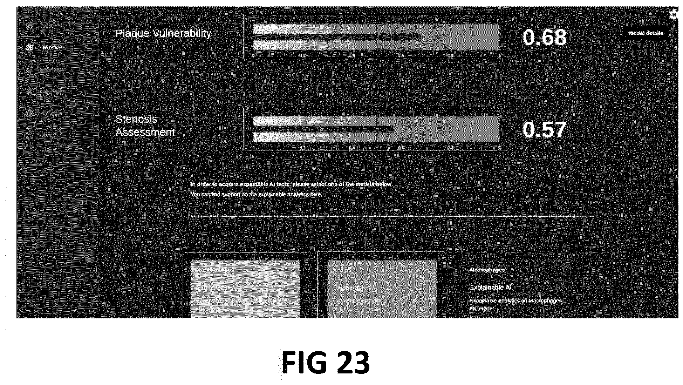

Fig. 23 shows a screenshot of the AI risk assessment platform.

DETAILED DESCRIPTION OF THE INVENTION

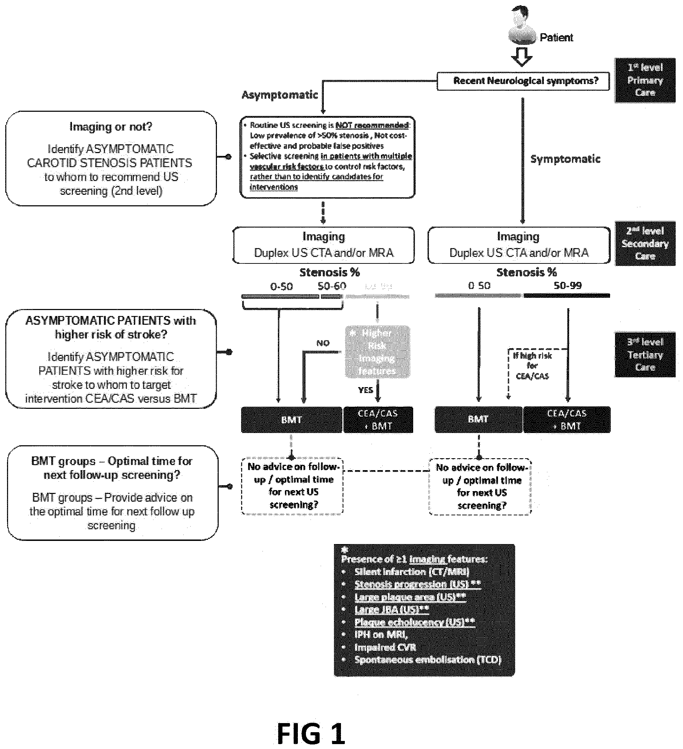

The management of carotid artery disease in the current clinical practice is based on the 2017 Clinical Practice Guidelines of the European Society for Vascular Surgery (ESVS) (Naylor AR et al., Eur J Vasc Endovasc Surg. 2018;55:3e81). The inventors have identified the following five open issues requiring suggestions for improvement (Figure 1): i) to help the General Practitioner to select patients who will benefit from carotid duplex and be candidate for intervention, ii) to help determine how frequent carotid stenosis should be followed once diagnosed, iii) to provide tools to predict progression, iv) to help multidisciplinary teams (MDTs) to select the patients who will benefit from Computed Tomography Angiography (CTA) and Magnetic Resonance Angiography (MRA) once carotid stenosis has been seen on Duplex, and v) to help MDTs select the patients who are at higher risk of cerebral event and might benefit from carotid intervention.

In the first stage, the Risk stratification tool utilizes non-imaging patient data such as demographics, clinical data and medication intake to provide input to a series of Machine Learning (ML) algorithms composing the following different models: i) Diagnosis of stenosis (threshold 50% stenosis); ii) Diagnosis of vulnerable plaque components (e.g. Intraplaque Hemorrhage (IPH), Lipid-Rich Necrotic Core (LRNC); iii) Evolution of silent brain lesions (SBLs); iv) Evolution of brain structural changes; v) Prognosis of cerebral outcomes/events (e.g. stoke, TIA, atrial fibrillation or SBLs).

If detailed imaging is available or required for improved stratification, then data from various imaging modalities (in depth analyses from carotid Duplex Ultrasound (US), carotid MRA and carotid CTA) are used to provide input to the imaging-based prediction models including:

- Plaque growth models.

- Blood flow models (plaque progression and event prediction models).

- Plaque rupture prediction models utilizing plaque burden and plaque composition features as well as biomechanical features and stresses that can be associated with previous or future plaque rupture symptoms or events.

Specifically, in the first step carotid image series from various imaging modalities such as US, MRI and CT are provided as input to the 3D reconstruction and plaque characterization module. The 3D reconstruction module provides as output the 3D arterial geometry while the plaque characterization module provides as output the plaque tissue composition (e.g., for MRI the presence of FC, LRNC, IPH and Calcification (Ca) or texture analysis features for the US). Plaque burden and plaque composition features such as plaque area, volume, normalized wall index (NWI), plaque echolucency, Juxtaluminal Black (hypoechoic) area (JBA) and plaque components area and volume are provided as analytic reports in the module's user interface.

In the next step, the 3D arterial geometry is used as input to the Blood flow modeling module to provide as output patient specific blood flow features such as blood velocities, blood pressure drop, wall shear stress (WSS) distribution, time-averaged wall shear stress (TAWSS), Oscillatory shear index (OSI) and Relative Residence Time (RRT) along the vessel's inner wall. The 3D arterial geometry and WSS values together with patient specific parameters including blood concentration of low density lipoproteins (LDL), High Density lipoproteins (HDL), monocytes, and T-lymphocytes, hypertension and diabetes status are used as input to the LDL (mass) transport modeling module to provide as output the respective concentrations accumulated in the carotid intima after their penetration across the endothelial layer. Then, the outcome of the LDL transport module is used as input to the Finite Element Method (FEM)-based plaque growth module. This module models the basic parameters of the atherosclerotic process by solving several differential equations and providing as output the concentrations of LDL, HDL, OxLDL, OxHDL, monocytes, microphages, Foam Cells, T-Cells, Cytokines, smooth muscle cells (SMCs), Collagen and plaque volume over time. The output of the module provides prediction of plaque volume evolution over time with an updated 3D arterial geometry. The new predicted 3D geometry is utilized by the blood flow modeling module to provide updated WSS distribution values.

In parallel, the initial 3D geometries of the vessel and individual plaque components from the 3D reconstruction and plaque characterization module, together with the WSS distribution from the blood flow module are provided as input to an agent-based modeling module. This module will provide stochastic modeling of the atherosclerotic process using behavioral rules for the agents. Agent concentrations including monocytes, macrophages, foam cells, T-lymphocytes, SMCs, cytokines, collagen, are provided as output in a time-depended manner predicting plaque volume and its components and an updated 3D geometry. At different time points, the updated geometry can be provided as input to the blood flow module to provide a hybrid model.

Initial (baseline) and predicted 3D vessel geometries are provided as input to the plaque rupture module to provide plaque burden and plaque composition features as well as biomechanical features and stresses (e.g. max tensile/principal Plaque Structural Stress (PSS) and WSS thresholds) that are collectively exploited to provide risk prediction for plaque rupture.

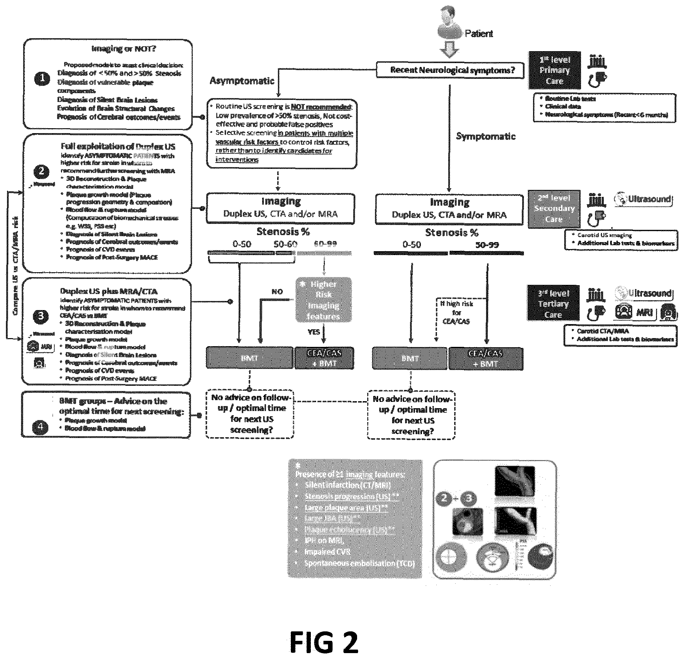

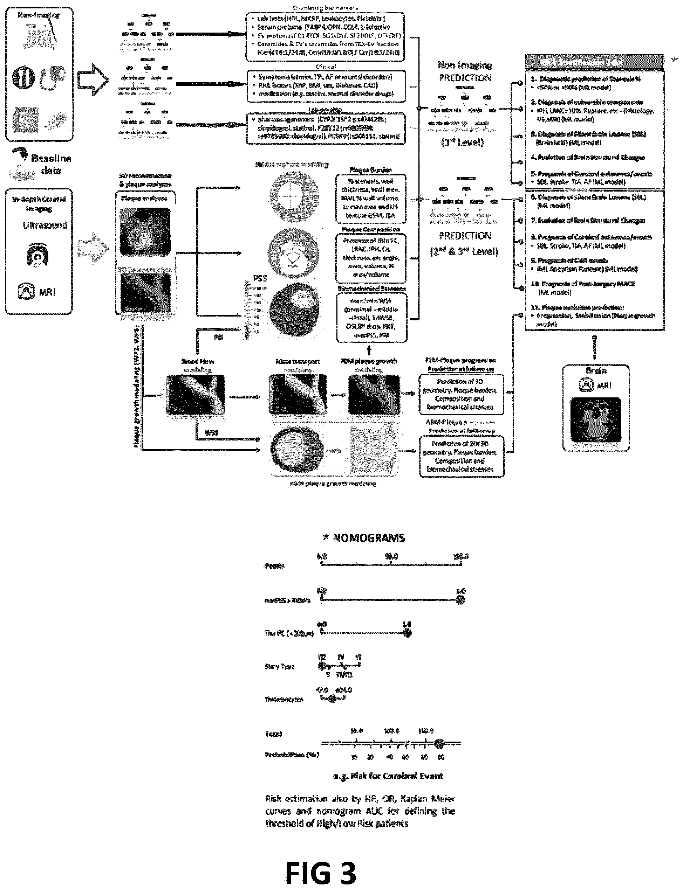

Features with high predictive ability from all models are integrated to develop the final risk stratification model to provide an overall risk estimation for plaque rupture, cerebral and cardiovascular disease outcomes. All model outputs are provided via knowledge presenting user interfaces and are validated by real patient data during the follow ups performed in the prospective clinical study. The contribution of the present invention to the standardof-care (SoC) following a 3-level approach is described in Figures 1-3. Figures 2 and 3 show in a graphical manner what is the added value for a new patient that will be stratified compared to the current guidelines and gaps depicted in Figure 1.

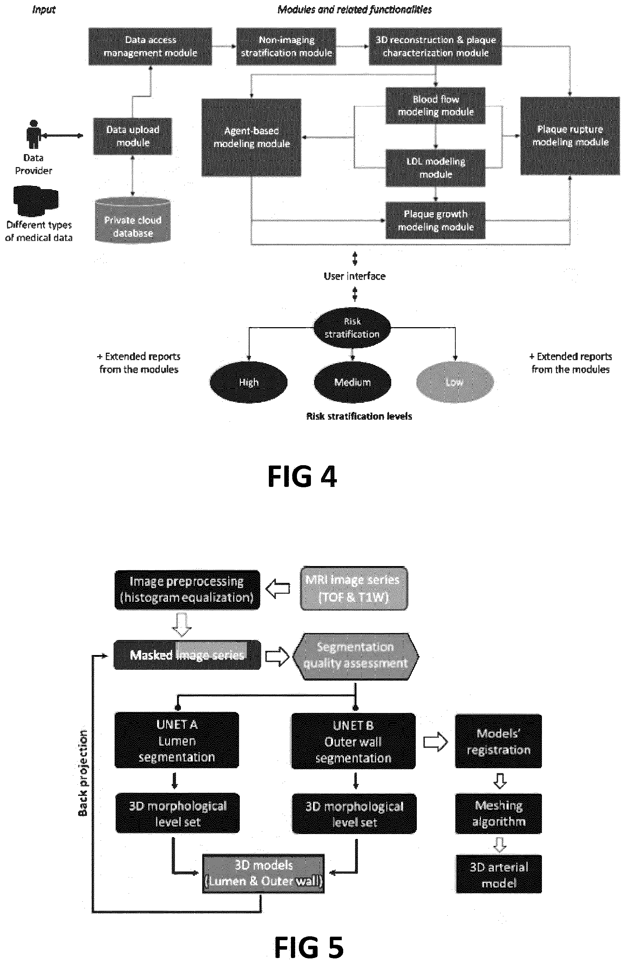

**Arterial Geometry Reconstruction and Plaque Characterization using MRI.** A novel methodology for reconstructing carotid arterial trees in an automated and real-time fashion using as input imaging modalities MRI series is presented. The proposed methodology utilizes texture-based image processing algorithms and machine learning models in a hybrid iterative framework and can accurately provide 3D meshed models of the lumen and the outer arterial wall, as evidenced by the validation study reported in the relevant section. From the systemic point of view, the MRI reconstruction methodology is incorporated as the main functionality of the 3D Reconstruction and Plaque characterization module of the overall architecture shown in Figure 4. Specifically, the proposed methodology contributes to the state-of-the-art approaches by training and deploying a deep learning model (U-net) for the image segmentation phase and utilizing an iterative approach according to which the produced model is refined over several iterations, aiming to reach certain quality criteria, based on the anatomic properties of the specific body area. The overall methodology is presented in Figure 5.

*Image segmentation model.* One of the most important components of the methodology described in the previous section is the segmentation component, which is used to isolate the pixels of interest from each MRI series (lumen from the Time-of-Flight (TOF) series and outer arterial wall from the T1W (T1-weighted) image series). For this, the U-net model has been utilized, as it has shown remarkable results on medical image segmentation tasks. The goal of this architecture is to produce competitive segmentation results given a relatively small quantity of training data.

*Training dataset.* To train the U-Net model, a set of MRI examinations has been collected. MRI examination was performed in 29 patients at baseline, enrolled in a prospective clinical study at the Technical University of Munich using a 1.5-T whole-body system (Signa HDx, GE Healthcare, Waukesha, WI, USA) with a bilateral four-channel phased-array carotid coil (Machnet BV, Eelde, NL). All patients gave informed written consent, and the study protocol was approved by the local regional ethics committee. The acquisition parameters for the MRI sequences were as follows: (i) TOF images: repetition time: 23 ms, effective echo time: 3.2 ms, field of view [FOV]: 160 mm, section thickness: 1 mm; (ii) fast-spin echo double-inversion recovery prepared sequences (T1W): repetition time: 1428.57 ms, effective echo time: 7.672 ms, FOV: 100 mm, section thickness: 3 mm; The acquired data were stored in DICOM format.

*Training dataset preparation.* Two specialists annotated the luminal wall on the TOF frames and the outer arterial wall on the T1W frames. Based on these annotations, masked images were created which were used as input for the training phase of the U-net models (one model for segmenting the lumen and one model for segmenting the outer arterial wall). This process resulted in two datasets, one for the luminal wall with 29 patients $\times$ 100 (TOF frames) $\times$ 100 $\times$ 100 pixels and one for the outer arterial wall with 29 patients $\times$ 10 (T1W frames) $\times$ 100 $\times$ 100 pixels. This deep neural network was implemented with PyTorch functional API. Output from the network is a 100 $\times$ 100 pixels image which represents the segmented image. The binary cross-entropy loss function was selected.

*Segmentation Results.* The parameters selected for each model component are the following: rectified linear unit (ReLU) activation function (for all layers but last), sigmoid activation function (for the last layer), He distribution for the kernel, Adam optimization algorithm. The two U-Net models have been trained for 7 epochs, where 300 batches (steps) per epoch were applied. As far as the loss function is concerned, the binary cross-entropy function has been used.

$$H_p(q) = -\frac{1}{N}\sum_{i=1}^{n} y_i \log\big(p(y_i)\big) + (1 - y_i)\log\big(1 - p(y_i)\big). \quad (1)$$

The estimated parameters for the model training phase were accuracy and F1 score, which is calculated through the formula (2):

$$F_1 = \left(\frac{2}{\frac{1}{recall} + \frac{1}{precision}}\right). \quad (2)$$

*Iterative process.* The cloud point of the previous procedure is back projected on the initial frames. Then, a connected components calculation algorithm is applied, aiming to detect on each frame all the remaining contours. Based on the anatomical features of the specific area, one would expect to have one object before the bifurcation, two objects just after the bifurcation and three to four objects 10mm above. When these criteria aren't met, the algorithm masks the frames based on the latest received level set and feeds models A and B again, aiming to refine the segmentation. This process continues until an anatomically valid model is produced, or after ten iterations.

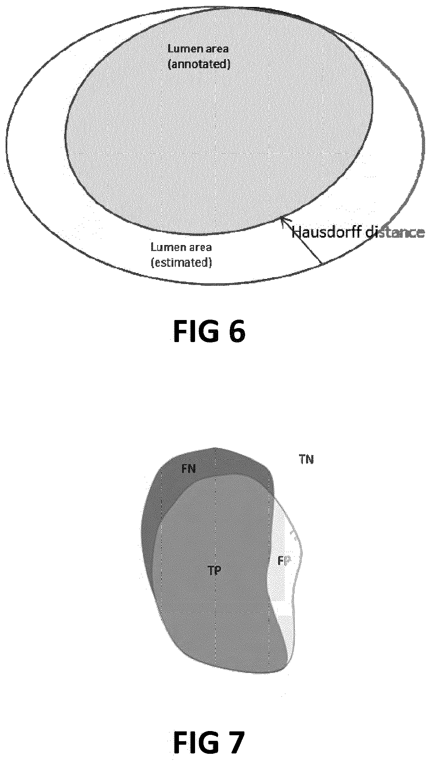

*Validation study.* Aiming to validate the 3D reconstructed models derived from the previously described methodology, a dataset of annotated MRI series has been collected. The annotated dataset was created by two experts, who annotated the dataset once. The validation dataset had no common data with the training dataset of the U-Net models. A validation study on both the luminal and the outer arterial wall has been performed, in order to assess the accuracy of the proposed scheme. For this, 2871 TOF and 981 T1W baseline MRI sequences from 68 patients were annotated by two specialists. On each frame, the specialist's annotation was treated as the ground truth and the area, the perimeter, the William Index, the Dice Coefficient and the Hausdorff distance were calculated. Figures 6 and 7 present the validation metrics in detail. Dice Coefficient is calculated through the formula (3):

$$D = \frac{2 * overlapping\,area(A, B)}{area(A) + area(B)} = \frac{2 * TP}{FN + 2TP + FP} \quad (3)$$

while the William Index through the formula (4):

$$WI = \frac{\frac{1}{n}\sum_{j=1}^{n}\frac{1}{D_{0,j}}}{\frac{2}{n(n-1)}\sum_{j}\sum_{j'\ne j}\frac{1}{D_{j,j'}}} \quad (4)$$

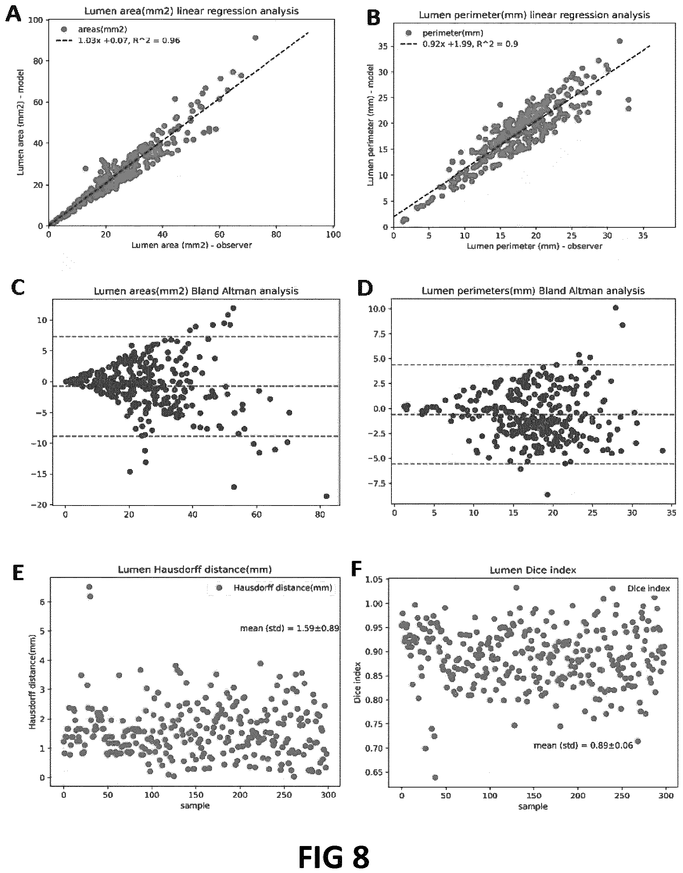

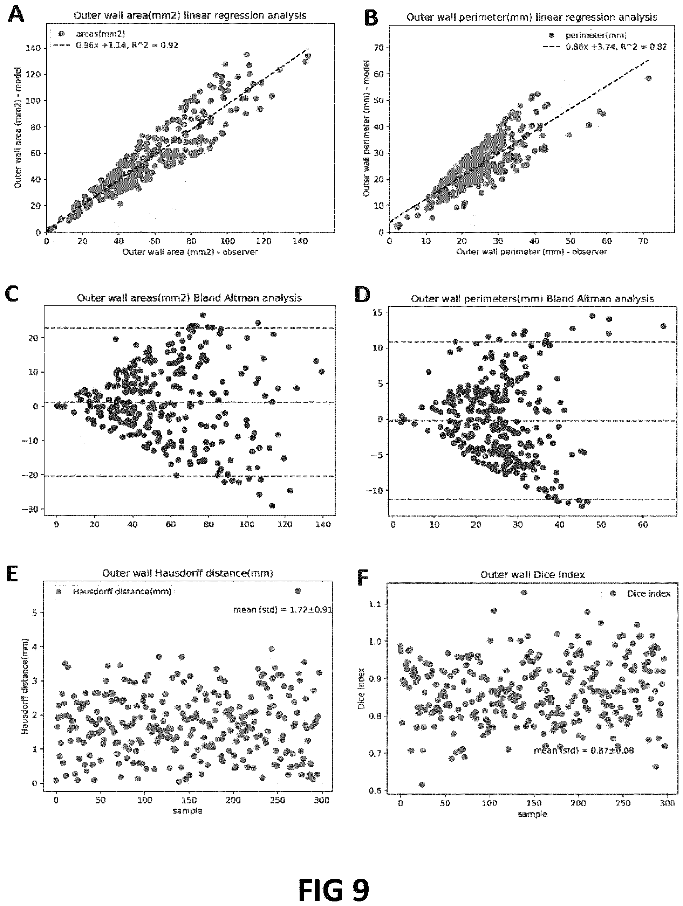

The results from the validation study are shown in Figures 8 and 9.

The prior art approaches for carotid artery MRI image segmentation mainly involve active contours (with various objective functions, like snakes or level sets) both in 2D and in 3D domains and ML approaches, which are produced by training different models (NNs, SVMs, etc.) with annotated data. The disclosed methodology contributes beyond the state-of-the-art by 1. Training and deploying a deep learning model (U-net) for the image segmentation phase, 2. Utilizing an iterative approach, according to which the produced model is refined over several iterations, aiming to reach certain quality criteria, based on the anatomic properties of the specific body area, and 3. Validating the proposed models over an extensive dataset of ground truth annotated dataset.

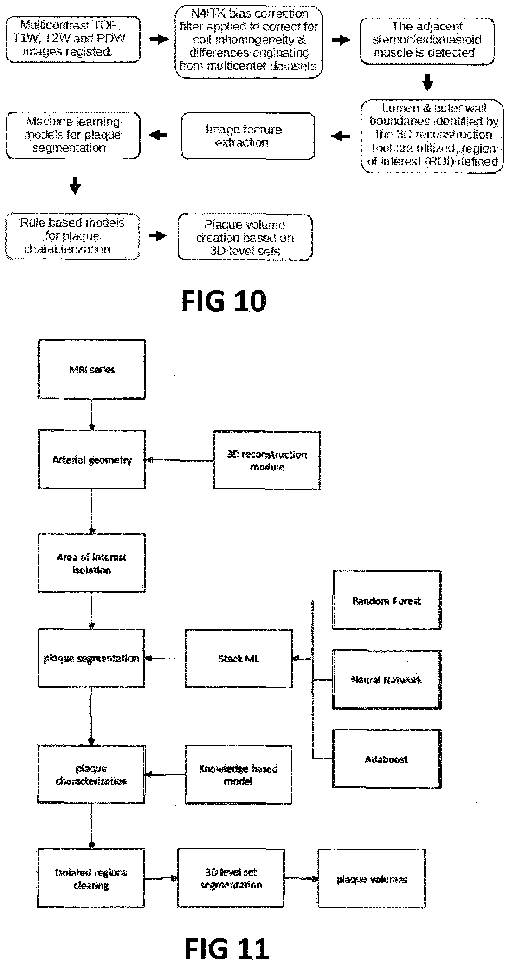

**Plaque Characterization using MRI modalities.** In order to refine the produced 3D artery model, information related to the plaque tissue components which produce the stenosis of the diseased arteries was integrated into the model. To this end, hybrid models of image processing methods together with supervised ML techniques are explored, to improve plaque type identification of the carotid arteries. The methodology exploits TOF, T1W, T2 weighted (T2W) and Proton Density weighted (PDW) MRI for more accurate identification of LRNC, IPH, calcification and fibrous tissue including thin or ruptured fibrous cap. The proposed methodology mimics that of the manual review and includes the steps presented in Figure 10.

*Ground truth datasets.* In order to support the aforementioned methodology, a dataset to act as ground truth for the ML models is required, the aim of the ML models being to detect and segment the plaque regions on every MRI frame. For this, two experts annotated once a dataset obtained from a total of 91 patients at baseline which have been enrolled in the prospective clinical study. For each case, approximately 18 frames per series have been annotated (TOF, T1W, T2W and PDW) using an annotation software tool. Lumen and outer wall border as well as plaque areas (calcifications, hemorrhages, fibrous cap, lipid cores) have been annotated on specific pre-defined image series. This process resulted in a dataset of 2421 annotated images.

*Supervised learning.* In the supervised setting an algorithm is trained with a training set in which every example has a ground truth. Since the goal is to perform pixel-wise segmentation, every pixel is an example that needs to be evaluated by the algorithm. It is thus also necessary to have a ground truth for each pixel (pixel-wise annotations). In recent years a new class of algorithms became increasingly popular: The supervised learning method used was Convolutional Neural Networks (CNNs). Other methods that may also be used are Linear Discriminant Analysis, Support Vector Machines, Random Forest and Neural Networks. For the ML methods only the features from the region between the lumen and vessel wall are evaluated. Per sequence the following features were obtained: the normalized MRI intensity values, scale space features such as gradient magnitude, Laplacian on three different scales, distance from lumen, distance from outer wall, texture features such as: local range, local entropy, local standard deviation, and local binary patterns. From the vessel segmentations the distance to the vessel wall and lumen were calculated as well as the multiplication between these two.

*Evaluating Performance.* Evaluating the performance of the classifiers is not a trivial problem. Especially in the medical field where the data sets are often imbalanced. Diseased tissue, the target class, is often a minority class compared to healthy tissue, the background class. However, the background class (fibrous tissue) still contains a vast majority of pixels. In such imbalanced data classifying every example to the majority class can result in an overall accuracy over 90%. For this reason, metrics are calculated over all the classes individually and other metrics are used to evaluate the performance of the algorithms on the imbalanced data set. All the metrics are derived from the confusion matrix. The metrics used are:

1. Accuracy: the proportion of true positives (TP) and true negatives (TN) to the total number of pixels for that class. It is the percentage of correctly classified pixels.

2. Sensitivity: also called true positive rate, recall or probability of detection. It is a measure to indicate the proportion of positives that are correctly identified as positives. Thus, for example the proportion of calcification that is classified as calcification.

3. Specificity: also called the true negative rate is the proportion of negatives that are correctly classified as negatives.

4. Precision: the proportion of true positives compared to the true positives and false positives.

5. F1-score: the F1-score or Sorensen-Dice index or the Dice Similarity Coefficient is the harmonic mean of the precision and sensitivity. It is a common metric for evaluating segmentation results.

$$F1 = \frac{2TP}{2TP + FP + FN} \quad (5)$$

6. Area under Curve (AUC) or area under the receiver operating characteristic curve (ROC curve). In the ROC curve the true and false positive rate are plotted for all possible decision thresholds. The ROC shows results for different decision thresholds and thus allows for additional optimization for this threshold. Thus the AUC combines sensitivity and specificity in a single metric. Random predictions would result in an AUC of 0.5 and the line x = y. AUC can be used to filter out models that are representative but not discriminative.

7. Cohen's Kappa: The intraclass correlation coefficient is a generalization of Cohen's kappa. Where ICC is used for measurements in the continuous domain kappa is used in the categorical domain. It estimates the agreement between observers with the consideration of agreement by chance.

$$k = 1 - \frac{1 - p_0}{1 - p_e} \quad (6)$$

where $p_0$ is the relative observed agreement (accuracy) among observers and where $pe$ is the hypothetical probability of agreement by chance. Cohen's kappa is often interpreted based on arbitrary chosen ranges, the most popular being: no agreement $\kappa \leq 0$, slight agreement 0-0.20, fair 0.21-0.40, moderate 0.41-0.60, substantial 0.61-0.80, and (almost) perfect agreement 0.81-1.00.

Figure 11 summarizes the detailed proposed methodology for plaque characterization. The first step of the proposed methodology invokes the MRI 3D reconstruction module, which provides as input the geometrical characteristics of the vessel. More specifically, the borders of the lumen and the outer wall are calculated and provided as input to the plaque characterization module. Using these borders, the area between them is isolated and passed as input to the next step. The next step involves the segmentation and characterization of the plaque regions. This problem is treated as a four-class classification problem, where the first class refers to "healthy" (non-plaque) pixels, the second class to pixels depicting calcified areas, the third class to pixels depicting fibrotic areas and the fourth class to pixels depicting lipid cores. The training dataset described in the previous section is utilized, to train ML algorithms. The most important features calculated for the supervised learning approach are presented below, however additional features may also be used.

1. Image gradient (magnitude): The gradient of an image is a vector of its partials derivatives.

$$|\nabla I(x,y)| = \left| \begin{matrix} \frac{dI}{dx} \\ \frac{dI}{dy} \end{matrix} \right| = \sqrt{\left(\left(\frac{dI}{dx}\right)\right)^2 + \left(\left(\frac{dI}{dy}\right)\right)^2} \quad (7)$$

where $dIdx$ is the derivative with respect to x (gradient in the x direction) and $dIdy$ is the derivative with respect to y (gradient in the y direction).

2. Laplacian Image: The Laplacian $L(x,y)$ of an image with pixel intensity values $I(x,y)$ is given by:

$$L(x,y) = \frac{d^2 I}{dx^2} + \frac{d^2 I}{dy^2}. \quad (8)$$

3. Local range:

$$LR(x,y) = MAX(I(x,y)) - MIN(I(x,y)) \quad (9)$$

where each output pixel contains the range value (maximum value - minimum value) of the 3-by-3 neighborhood around the corresponding pixel in the input image $I(x,y)$.

4. Local entropy:

$$H(I) = \sum_i h_I(i) log \frac{N}{h_I(i)} \qquad (10)$$

where $h_I(i)$ denotes the histogram entry of intensity value $i$ in image $I$ and $N$ is the total number of pixels of $I$.

5. Euclidean distance:

$$D(I(x,y)) = \sqrt{x^2 + y^2} \qquad (11)$$

6. Standard deviation:

$$SD(I(x,y)) = \sqrt{\frac{1}{nm-1} \sum_{(r,c) \in W} \left( g(r,c) - \frac{1}{nm-1} \sum_{(r,c) \in W} g(r,c) \right)^2} \qquad (12)$$

where $n,m$ refer to the neighborhood on which the filter is applied, and $W$ is the neighborhood size.

*Model training.* Using the features detailed in the previous section, a set of ML models has been tested, to evaluate their performance. It is reminded that the objective of this model is to classify all pixel among lumen and outer border as either "healthy", or "diseased". A large scale of experiments has been conducted, aiming to come up with the most appropriate model for the specific dataset. The tested ML models included linear and polynomial kernels on SVMs, decision trees, random forest, Adaboost and Stacking approaches, as well as kNN and neural networks, however, kNN outperforms all other approaches substantially.

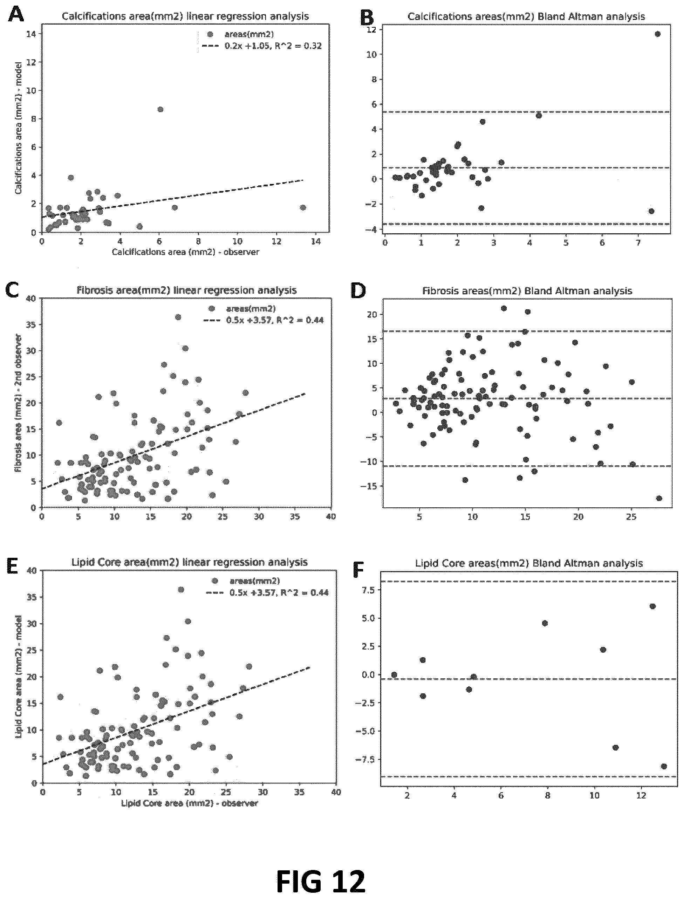

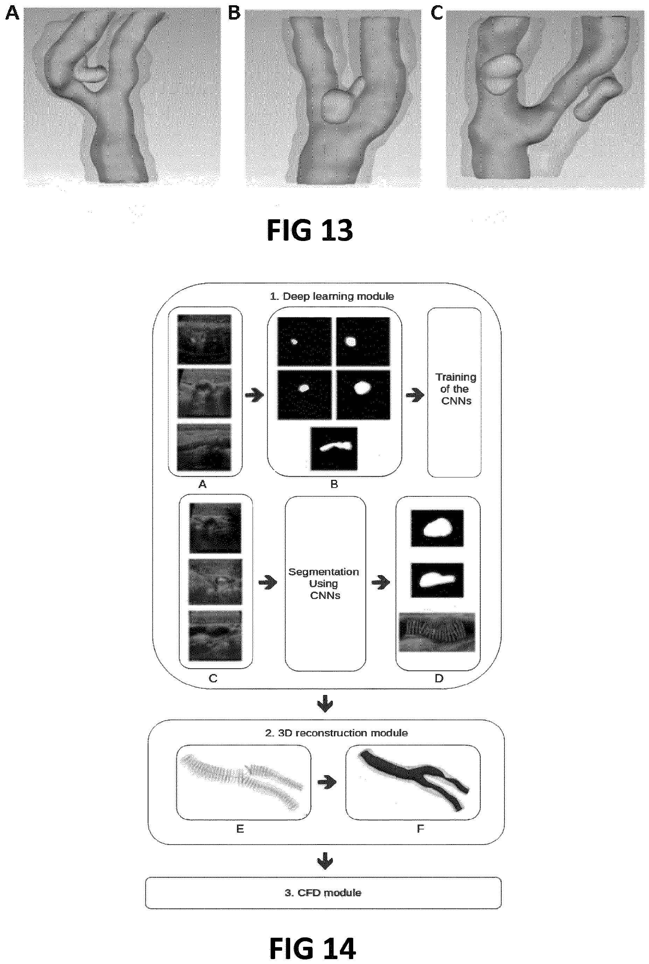

*Validation Results.* Two cases of diseased arteries that were excluded from the training set of the ML models were used to evaluate the proposed methodology. For this, after creating the 3D models of the arterial trees, the kNN classifier was applied and the segmented regions have been extracted. For the next step, the trained model has been applied, and the regions of different plaque types have been extracted. The obtained results have been compared with the ground truth dataset (randomly selecting among the two observers), and the results are presented in Figure 12. The acquired metrics from the plaque detection and characterization module are: (i) Plaque type, (ii) Plaque site, (iii) Plaque volume, (iv) Plaque distance from lumen and outer wall. Figure 13 presents some sample case studies, where the lumen, the outer wall and the plaque components are presented.

To summarize, plaque characterization is a challenging research field, open for more than twenty years now. Many works have been proposed from both academia and industry, all aiming to facilitate the workflow of radiologists and medical doctors. Yet, all the proposed algorithms and methodologies are usually "tied" to the MRI acquisition protocol, the characteristics of the MRI scanner and other in-house protocols. This heterogeneous environment prevents the direct comparison of different approaches, as they are related to the quality of the MR image acquisition and the utilized MRI series. MRI examinations were provided from four different centers, allowing the design of both data driven approaches and knowledge-based models which are more generalized and more widely applied than most reported similar works. Finally, the validation scheme on the collected dataset provides a robust method with the capacity to be applied to a wide range of medical centers.

**Arterial Geometry Reconstruction and Plaque Characterization using Ultrasound.** Carotid artery stenosis (CAS) is a major consequence of carotid artery disease and its early detection is very important because if not adequately treated, it may potentially have deteriorating effects. Visualizing and analyzing the morphological structure of carotid bifurcations are important for understanding the etiology of carotid atherosclerosis, which is a major cause of stroke and TIA. US is usually the initial recommended CAS diagnostic examination. For delineation of vasculatures in the carotid artery, US examinations have been widely employed as they constitute a noninvasive procedure without ionizing radiation. 2D US imaging is a widely available tool for the diagnosis and management of carotid plaque atherosclerosis. However, conventional 2D US imaging has technical limitations in observing the complicated 3D shapes and asymmetric vasodilation of bifurcations. Also, 2D ultrasound can only provide a limited number of views (longitudinal and transverse) the quality of which and information contained within them can vary since it is an operator-dependent technique. Moreover, once the 2D slices have been captured, images of other planes can no longer be extracted. This may affect the accuracy of plaque characterization since information is only sampled from one section, which is then assumed to be representative of the entire plaque. Furthermore, there is accumulating evidence that plaque composition, morphology and size may significantly impact plaque vulnerability. The 2D approach may not be sufficient to evaluate these features accurately for entire plaque volumes since many carotid plaques have variable compositions with some of their components being more echo-lucent than others. Thus, in heterogeneous carotid plaques, 2D US imaging may not provide an accurate assessment of the composition or size, with obvious implications in risk stratification. The development of angio-MRI and angio-CT introduced a new era in volumetric vascular imaging. Following the use of highly sophisticated reconstruction software and the acquisition of images containing volume information in three dimensions, the visualization of vascular structures in different planes from different angles is possible. Thus, a more holistic evaluation of the plaque properties can be achieved. Nevertheless, these modalities have their own limitations including limited accessibility, increased cost, exposure to radiation, a more invasive nature, increased patient inconvenience and contraindication in patients with contrast-allergy or renal impairment. The characterization of carotid plaques using non-invasive and reproducible 3D imaging techniques will potentially improve investigations into the changes of surface morphology, plaque geometry, and plaque distribution and these can provide important information about the effects of anti-atherosclerotic therapies. The US examination can be used to measure wall thickness and blood velocities in patients. Additional parameters that cannot be directly measured can be obtained using numerical simulations, more precisely computational fluid dynamics (CFD) methods. The simulations can be performed using idealized carotid bifurcation, but in order to determine the correct patient-specific diagnosis that also considers the individual anatomy of the particular patient, it is necessary to perform simulations using a patient-specific geometry. According to the invention, images obtained using the US examinations are used to adapt the generalized carotid bifurcation model to the specific patient.

*Methodology.* The scheme of the entire methodology within the US image processing module disclosed herein is shown in Figure 14 and explained below. US images used as the basis for the whole reconstruction module are shown in Figure 14A. These images are annotated and preprocessed as shown in Figure 14B. These pairs of original and annotated images are used for the training of the CNNs. Then, a specific set of US images (Figure 14C) is used to extract the segments of the CA by using the trained models (Figure 14D). The information obtained in the deep learning module is further directed to the reconstruction module, where the relevant shapes of the carotid bifurcation are created (Figure 14E), in order to obtain the finite element mesh of the reconstructed geometry (Figure 14F). Finally, the finite element mesh is ready to be used within the CFD module for the simulations of blood flow and plaque progression.

*Image preprocessing.* The first step of the preprocessing is the automated isolation of the image region which contains the arterial tree under reconstruction. This is performed by selecting a static 512x512 pixels window for both arterial models, left and right. Special attention is paid to the window coordinates in order for the whole arterial tree to be visible in the region. After this step, all images are labeled thus creating two datasets with labeled regions, one for the lumen and one for the wall. Images in the dataset are extremely diversified in terms of colors, brightness/contrast, frames and tables. Significantly lower brightness/contrast of the figure caused an overall dark appearance of the figure, where dark zones occupy a lot more space than on other figures. Also, some figures have frames containing various information unlike others, and even the spaciousness of the figures is different, i.e. they occupy less space on the figures. Additionally, it can be observed that some figures have tables containing various parameters on the right side, while others do not, and that color scales can be different.

*Image segmentation methodology.* The automatic carotid artery (lumen and wall) segmentation has been done using FCN-8s, SegNet and U-Net based deep convolutional networks. Beside the original versions of these architectures, the U-Net and SegNet networks were modified from the aspect of depth in order to test their capabilities to recognize the regions of interest. Finally, the best results were obtained by using the modified version of U-Net CNN, which has two additional blocks in both encoder and decoder. Each block in encoder has two convolutional layers with $3\times3$ filters, followed by $2\times2$ max pooling. Encoder blocks produce output with respectively 24, 64, 128, 256, 512 and 768 channels. In each decoder block, $2\times2$ upconvolution and skip connection are followed by three more convolutional layers with $3\times3$ filters, and the last decoder block produces the segmentation mask with $1\times1$ convolution and sigmoid activation function. All convolutional layers are padded so that the resulting activation map preserves the same height and width. In this way, the resulting segmentation map has the same resolution as the input image. The variant U-Net uses batch normalization after each convolutional layer and all batch normalization layers are followed by a ReLU activation. The model is trained with a combination of binary cross-entropy and soft dice coefficient (Anbeek P et al., Neuroimage. 2005;27(4):795-804) as a loss function, which is expressed as:

$$Loss = binary\_crossentropy(y_{true}, y_{pred}) + 1 - dice\_coeff(y_{true}, y_{pred}) \qquad (13)$$

where $y_{pred}$ and $y_{true}$ denote the flattened predicted probabilities and the flattened ground truths of the image.

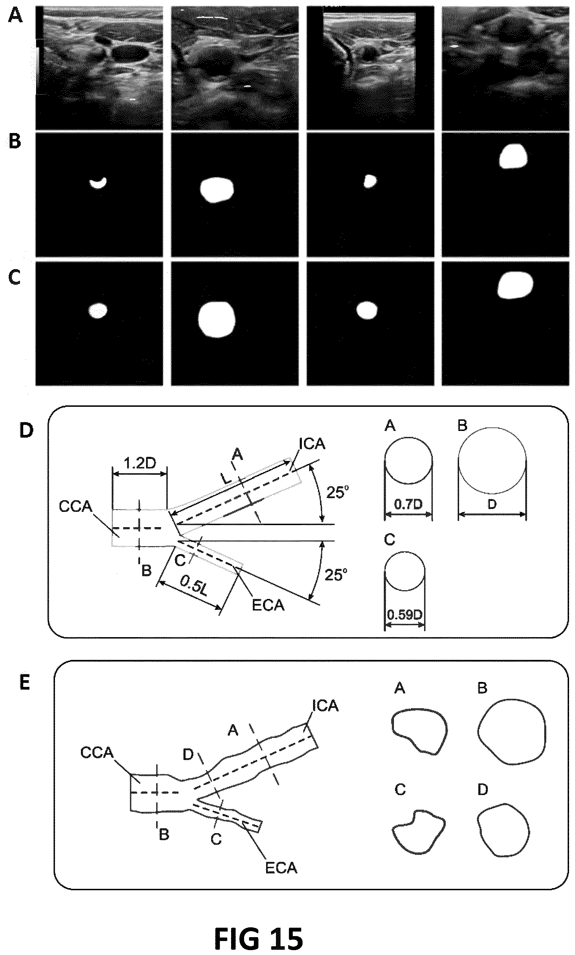

*Experimental setup.* Dataset consists of the US images of 39 patients. All subfolders corresponding to the patients are randomly divided into training, validation and testing sets by a ratio of 8:1:1 at the carotid artery level (either for the left or for the right arterial model). The dataset contained a total of 179 original images and was further augmented for training purposes. Random selection is important if we want to ensure model robustness when the images from completely new dataset (new slices obtained from another patient) are provided as the model input. This is also necessary and important because the carotid arteries are not symmetric about neither *x*-axis nor *y*-axis. Also, the patient's age, weight and height are also important factors which influence the final US images look. It is known that U-Net works very well in combination with data augmentation, and that it can be trained on small datasets. The number of training images has been further increased using data augmentation techniques because of the small size of the training set. Figure 15 shows examples of the original (Figure 15A) and labeled images for lumen (Figure 15B) and wall (Figure 15C).

*Image segmentation results for the lumen and wall - transversal US images.* In this case, binary classification task for image segmentation was considered. Three common classification metrics are considered for quantitative evaluation, precision (P), recall (R), and F1-score coefficient. The results for the test set for lumen and wall are shown in Table 1 and Table 2, respectively.

Table 1. U-Net results on test dataset for lumen.

| Precision | Recall | Dice coefficient (F1-score) |
|-----------|--------|-----------------------------|
| 0.89 | 0.78 | 0.83 |

Table 2. U-Net results on test dataset for wall.

| Precision | Recall | Dice coefficient (F1-score) |
|-----------|--------|-----------------------------|
| 0.82 | 0.91 | 0.85 |

All the tests were performed on GIGABYTE NVIDIA GeForce GTX 1080 Ti 11GB, GDDR5X, 352bit. Python V3.6.7 is used as the programming language and related code is implemented using Keras framework, which regards Tensorflow framework as the backend.

*Model deployment.* A deployment procedure of the developed deep learning models for US image segmentation task of carotid artery was realized by using FLASK and Docker technologies. This methodology can be applied for any other dataset of US images. In the first step, two separate FLASK applications are developed, one for the transversal US images projection and the second one for the longitudinal US images of carotid artery. Then, two Docker images (python:3.7.10-slim-stretch) are developed, one image is determined only for the transversal and the second one only for the longitudinal carotid artery projection. For reconstruction of the carotid artery, a set of python scripts is applied through API. These scripts are used in order to extract only desired lines in both longitudinal and transversal images since many images contain artifacts which need to be excluded. This is done using multiple filters, contour detections and thresholding methods as well as automatic image editing. At the end, for transversal images, API outputs a single textual file containing either lumen or wall coordinates (depending on which API is called), and for longitudinal images, API outputs two textual files containing coordinates of upper and lower boundary lines of either lumen or wall of common carotid artery (CCA) depending on which API is called.

*3D reconstruction of the carotid artery.* The 3D reconstruction of patient-specific carotid artery is performed using the available clinical imaging data for the particular patient. A main problem with the patient data set is the limited number of 2D transversal cuts that are available. In order to overcome this problem, the generic model proposed in literature was used as the basis. This basis is then adapted to the specific patient, by including the available data in the geometry. The generalized model, illustrated in Figure 15D, was defined according to data presented in (Kerktold K et al, J. Biomech. 1991;24(6):409-420). The improved model disclosed in shown in Figure 15E. The longitudinal cut of the ICA is used to extract the centerline of the internal carotid artery ICA and the diameters in this segment. The arterial wall is also reconstructed using the available clinical data, in combination with generic data presented in literature, using the same approach used for lumen. The transversal cuts of the CCA, external carotid artery (ECA) and ICA branches (lines A to D in Figure 15E) are used to define the shapes of the cross-sections of these branches. With the improved methodology disclosed herein, the longitudinal US images contained the whole carotid bifurcation. Hence the segmented data included lines of lumen and wall for all three branches. These lines are then used to define the shapes of all three branches, as illustrated in Figure 15E. The parts of the model marked with a blue square are the ones adapted to the particular patient, thus the whole model is adapted to the particular patient. The lengths of the branches and their positions in space are also now patient-specific and not generic.

The 3D reconstruction process is performed using the following algorithm. First, the extracted segments are obtained using the procedure explained in Figure 14D. The obtained curves are smoothed, converted to nonuniform B-spline curves and projected along the extracted centerlines (Figure 14E). At the end, the 3D FE mesh is generated (Figure 14F). Individual branches (CCA, ICA and ECA) are modeled as tube-like surfaces. For each branch, a parameterized $\vec{c}^i(t)$ centerline is defined (where index i represents the branch and CCA is denoted by 1, ICA by 2 and ECA by 3):

$$\vec{c}^{i}(t) = \sum_{i=1}^{q} \mathbf{x}_i N_{i,k}(t) \qquad (14)$$

where $0 \le i \le 1$ and $2 \le k \le i+1$. In Eq. (14) the control points of the centerline are defined with $x_i$, and $N_{i,k}$ denotes the k-th order basis functions, that are calculated according to the Cox-de Boor recursive algorithm:

$$N_{i,0}(t) = \begin{cases} 1 & if \ s_i \le t < s_{i+1} \\ 0 & otherwise \end{cases}$$

$$N_{i,k}(t) = \frac{(t - s_i)N_{i,k-1}(t)}{s_{i+k-1} - s_i} + \frac{(s_{i+k} - t)N_{i+1,k-1}(t)}{s_{i+k} - s_{i+1}} \qquad (15)$$

where s represents the knot vector calculated using the Chord length algorithm. The trihedron of the centerline $\vec{c}^i(t)$ is defined using the Frenet-Serret formulas (Vukicevic AM et al., Med. Biol. Eng. Comput., 2014;52:539-556). The trihedron is defined via the curve's tangent $\vec{T}^i(t)$, normal $\vec{N}^i(t)$ and binormal $\vec{B}^i(t)$. The tube-like surface for each branch is defined with cross-sections positioned along the parameterized centerline. The cross-sections are either circles with extracted diameters, or the extracted segments. In both cases, the points of the cross-sections are projected onto the trihedron normal-binormal plane of each centerline to obtain the patches of the surface. In order to ensure the regularity of points and to prevent twisting of individual patches, all points were converted to polar coordinates in normal-binormal plane and sorted in circular direction. These patches in 3D space are used for non-uniform rational basis spline (NURBS) surface representation that can be defined as:

$$\bar{S}^i(u, v) = \sum_{i=1}^{q} \sum_{j=1}^{w} \mathbf{B}_{i,j} N_{i,k}(u) M_{j,l}(v) \qquad (16)$$

where $0 \le u \le 1$ and $0 \le v \le 1$. In Eq. (16) the homogenous points of the control net polygon are defined with $B_{i,j}$ and $N_{i,k}$, and $M_{j,l}$ denote the k-th and l-th order basis functions, respectively. The basic functions are calculated using Eq. (15). Using the parameterized vessel centerline $\vec{c}^i(t)$ and surface $\vec{S}^i(u, v)$, it is possible to perform the discretization of the vessel. The introduction of the parameterization enabled to control the meshing process by defining the density of nodes in the mesh in both longitudinal (u) and circular (v) directions. Details of the meshing procedure are described in (Vukicevic AM et al., Sci Rep. 2018;8:1711). The main idea is to decompose the branches of the carotid artery into connected hexahedra elements, which were composed of quadrilateral patches (that are actually the vessel cross-sections). Finally, the individual branches of the carotid artery are then connected into a bifurcation, following the procedure described in (Vukicevic AM et al., Sci Rep. 2018;8:1711). Within the 3D reconstruction process, the separation of plaque from the arterial wall was also performed. The inventors concluded that the plaque separation from the arterial wall should be performed such that a layer of the plaque should be present radially within the entire wall. This was ensured in the reconstruction software in two steps: 1) The location of the plaque along the longitudinal directions of all three branches was computationally determined, and 2) A layer of elements representing the plaque was extracted from the previously created finite element (FE) mesh in radial direction. The automatic calculation of the plaque in the longitudinal direction was performed such that the ratio of the wall and lumen diameters was calculated along this direction in several cross-sections. The cross-sections were performed at approximately 2 mm. The cross-sections that have this ratio larger than 15% are considered stenotic and in these cross-sections the plaque layer was separated. After separating the plaque from the arterial wall, the different plaque components can also be incorporated into the FE mesh. This is done by first performing the segmentation of plaque components from transversal US images and then by overlapping the FE mesh with the segmented data to mark the elements of the FE mesh that belong to the fibrous, lipid and calcified plaque.

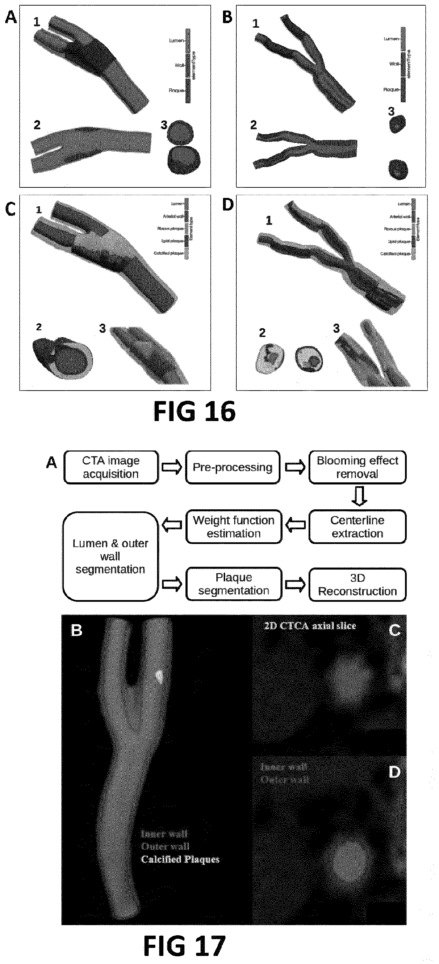

A comparison of a patient-specific reconstructed mesh using the generalized model and the improved approach disclosed herein shows that the shape of the ECA and CCA branches is no longer generic and straight, but adapted to the particular patient. Also, the exact orientation, i.e. the angle between the branches, is now patient-specific instead of generic like in the previous model. Thus, the changes introduced within the methodology provide a more detailed and realistic model of the carotid bifurcation. The geometries of the reconstructed models are shown in Figure 16 A and B. The whole mesh and two cross-sections are shown for each patient, in order to illustrate the shape of the transversal cross-sections reconstructed from US images and to show the automatically extracted location of plaque within arterial wall of the carotid bifurcation. The results of the 3D reconstruction including the separation of plaque components are shown in Figure 16 C and D for the same two representative patient-specific carotid bifurcations. The fibrous plaque is colored yellow, the lipid plaque is colored blue and the calcified plaque is colored green.

*Validation.* Two parameters were measured during US examination, the plaque length and the percentage of stenosis. The percentage of stenosis is measured on the ICA transversal cut, as the percentage of the current area of the lumen compared with the average area of the lumen for the ICA branch. The plaque length is determined from the ICA longitudinal image, as the length of the ICA where the stenosis is present. The values of these quantities are calculated for the reconstructed models to validate the segmentation and reconstruction modules of the proposed methodology. As it was already mentioned, the diameters of the ICA are calculated from the segmented longitudinal images. The diameters are measured approximately on every 2 mm. Overall 20 patients were considered for the validation of the methodology. There was a good correlation between percentage of stenosis obtained after reconstruction with clinical measurements, with the parameter of linear regression equal to $R2=0.7828$ and a good agreement between the two values can also be observed from the Bland-Altman plot. The mean difference of the results was 3.1404 (SD=4.3229). The corresponding limits of agreement were from - 5.3324 to 11.6133, with 95% confidence intervals of -8.85 to -1.8148 for the lower limit and 8.0957 to 15.1309 for the upper limit. Better correlation was obtained for the plaque length, with the parameter of linear regression equal to $R2=0.8851$. The mean difference of the results was 0.4629 (SD=2.1754). The corresponding limits of agreement were from -3.8009 to 4.7267, with 95% confidence intervals of -5.5711 to -2.0308 for the lower limit and 2.9566 to 6.4969 for the upper limit. The obtained good correlation of the results demonstrates the capabilities of the proposed methodology.

**Arterial Geometry Reconstruction and Plaque Characterization using Computed Tomography.** Manual segmentation of carotid arteries is a time consuming and tedious procedure and is highly dependent on the expert observer's ability to detect artery lumen borders. Thus, there is a strong need for the development of a computeraided segmentation procedure both for lumen detection and the atherosclerotic plaque detection and characterization. Thus, the inventors have developed a novel automated methodology for the 3D reconstruction of carotid arteries and the carotid atherosclerotic plaques using CTA. More specifically, the developed algorithm has as input the head and necks CTA images and provides as output the 3D geometry of the inner wall, the outer wall and the plaques of the carotid arteries. The algorithm requires minimal user interaction to provide the 3D carotid artery geometry. More specifically, the user imports the CTA images and annotates the starting and the ending point of the carotid artery that is going to be reconstructed.

*Methodology.* The overall pipeline methodology is shown in Figure 17A. An extended algorithm for the 3D reconstruction of carotid arteries atherosclerotic plaques using CTA imaging modality was developed and calibrated based on the CTA images quality, the CTA scanners and the acquisition dose protocols. The 3D reconstruction algorithm aims to segment the inner wall, the outer wall of the carotid arteries and to identify the calcified plaques (CP) and the noncalcified plaques (NCP) into the ROI. The utilized segmentation methodology is a 3D level set methodology as far as the inner wall, outer wall and CP segmentation is concerned, whereas the NCP pixels are identified based on dynamic thresholding technique. The main concept of the proposed methodology is that it is adapted in all acquisition dose protocols and CTA images derived by different CTA scanners. Overall, the proposed methodology includes different steps: 1) estimation of intensity membership functions for the inner wall, outer wall and CP, 2) carotid artery centerline extraction, 3) inner wall segmentation, 4) outer wall segmentation, 5) CP segmentation, 6) NCP segmentation and 7) 3D model construction. Briefly, three different sigmoidal membership functions are estimated for the three components (the inner wall, the outer wall and the CP). These membership functions aim to correspond its pixel to an inner wall, outer wall or CP pixel and are estimated in two different ways. The overall concept of these membership functions use is to adapt the intensity range for each component on the value of mean lumen intensity pixels. These membership functions are defined either based on fixed thresholds derived by the literature or based on threshold defined by the user after a calibration procedure. In the second step, a minimum cost path-based approach proposed by Metzt et al. (Medical physics. 2009;36:5568-5579) for the carotid artery centerline extraction is applied. In the third, fourth and fifth step a 3D level set approach using prior shapes is applied for the segmentation of the inner wall, the outer wall and the CP, whereas in the sixth step a dynamic threshold technique is implemented for the NCP segmentation. Finally, a triangulation methodology approach is applied for the 3D models construction. The proposed methodology is an extension of an already existing methodology for the 3D reconstruction of coronary arteries (Kigka VI et al., Biomed. Signal Process. Control. 2018;40:286-294) and is calibrated for the carotid arteries. The overall methodology has been incorporated into a software tool, which is easily used and requires minimum interaction with the user (only the starting and ending point of each vessel). The proposed methodology is able to provide not only carotid segment, but also carotid bifurcations which are high risk regions for atherosclerotic plaque growth. Figures 17B, C and D demonstrate (B) the 3D reconstruction of a carotid artery bifurcation, (C) the 2D CTCA slice without segmentation and (D) the 2D segmentation of the inner wall and outer wall.

*Results* & *Validation.* The aim of the validation process is to assess the accuracy and the quality of the proposed methodology, using as gold standard a medical expert's annotations. It is known that the lack of expert-annotated datasets remains one of the main challenges in medical image processing. Generating high-quality expert-derived annotations in CTA images is time-consuming and requires a specialized in CTA imaging field medical expert. More specifically, a medical expert annotated slice per slice of the axial CTA image the inner wall, the outer wall and both types of carotid atherosclerotic plaques, the CP and the NCP ones of 16 patients. Two different validation strategies were followed. In the first validation strategy, a validation procedure was implemented as far as the 2D segmentation is concerned, computing the DICE coefficient index and the Hausdorff distance. Reconstruction of carotid arteries, both for the left common artery (LCA) and the right common artery (RCA), was successfully obtained in 16 patients (dataset #1) and 12 patients (dataset #2). The results indicated that the DICE coefficient is 0.83 and 0.8, whereas the Hausdorff distance is 1.55 and 1.44, as far as the inner wall and outer wall detection is concerned, respectively in the dataset #1. For dataset #2, the evaluation metrics were evaluated for the calcified and the non-calcified plaques. More specifically, the DICE coefficient is 0.71 and 0.7, whereas the Hausdorff distance is 1.63 and 1.62, as far as the calcified and the non-calcified plaques are concerned, respectively. The respective evaluation metrics for the identification of CP is 0.71 and 1.63, whereas the Dice coefficient and the Hausdorff distance for the NCP are 0.7 and 1.55, respectively. In the second validation strategy, correlation analysis was implemented for the comparison of inner wall and outer wall area, derived by the automated proposed segmentation algorithm and these derived by manual expert annotation. Validation was performed on 650 inner wall slices and 500 outer wall slices and the Correlation coefficient for the inner wall and outer wall was 0.8 and 0.82, respectively.

The detection of both the inner and the outer wall of the carotid artery constitutes the novel aspect of the proposed methodology, since both layers have a clinical significance. Specifically, the degree of stenosis, calculated by the segmentation of the inner wall, is considered as one of the most significant geometric features of the carotid artery and its value contributes to the clinical decision and the patient management. Additionally, the quantification of the overall plaque burden region, computed by the detection of both the inner and outer wall, is a significant measurement as far as the carotid artery disease prediction and progression is concerned. Another advantage of the disclosed methodology is its incorporation in a user-friendly software tool, which provides the 2D segmentation in each 2D CT axial slice, the 3D rendering of both the inner and outer wall of the carotid arteries and the geometrical based metrics.

**Stratification based on non-imaging data.** The first level of care of risk stratification tool includes five different clinical problems and each one is developed using only non-imaging based data. Specifically, the clinical problems' outputs are: 1. Diagnosis of <50% and >50% Stenosis, 2. Diagnosis of vulnerable plaque components, 3. Diagnosis of SBLs, 4. Evolution of Brain Structural Changes, 5. Prognosis of Cerebral outcomes/events. Machine learning based models are trained for each clinical problem, using as input either retrospective datasets or the input dataset of the prospective clinical study. These datasets include demographics (sex, age), clinical characteristics (smoking, blood pressure, pulse rate etc.), medical history (Diabetes mellitus, hypertension, hypercholesterolemia, stroke, etc.), medication therapy, hematologic features (Hemoglobin, Hematocrit), lipid biomarkers (cholesterol, LDL-cholesterol, HDL-cholesterol, etc), inflammatory biomarkers (C-reactive protein, Osteoprotegerin, etc.), biochemical biomarkers (creatinin, urea, GFR, etc.), Imaging Color duplex ultrasonography (Degree of stenosis, etc), and MRI imaging data (Diffusion weighted imaging, acute ischemic brain lesions, intimal thickening, lipid or necrotic core, haemorrhage, calcification).

The first problem aims to identify individuals at high risk for the presence of at least one of the carotid arteries with CAS 50%. The development of these models is based on retrospective datasets, with different clinical views of input features. The detection of these individuals has been formulated as a multivariate 2 class classification problem based on the carotid DS, as it is defined by carotid US. High risk group (Class 1) included the individuals whose at least one of the carotid arteries (common, internal or external, left or right) has a DS above 50%, whereas low risk group (Class 0) included the individuals whose CAS were below 50%.

For the diagnosis of vulnerable plaque components, high risk plaques were defined based either on the histological composition of atherosclerotic plaques or on the presence of symptoms. The carotid artery disease risk stratification problem based on the vulnerability of plaques has been formulated as multivariate 2-class classification problem of the presence of high-risk plaques. In order to provide a multilevel characterization of an individual's status, five subproblems were defined depending on the datasets used: 1) composition of total collagen, 2) composition of SMCs, 3) composition of lipid, 4) composition of neutrophils, 5) composition of macrophages.

The aim of the third problem is to identify those individuals with SBLs. An expert neuroradiologist annotates the cerebral MRI images at the different time steps of the clinical study. During the analysis of brain MRI images, it was observed that individuals with the presence of SBL may be asymptomatic. Thus, the main idea of this third problem is to identify those individuals whose brain lesions are related to the carotid territory and are of high risk for a cerebral event.

The aim of the fourth problem is to identify those individuals who have a progression in their brain lesions, either these lesions are symptomatic or asymptomatic. The annotation of brain MRI images has to be performed at the baseline step and the follow-up steps.

The fifth problem aims to identify the patients at high risk for a future cerebral event. For the development of this machine learning model, the presence of TIA, stroke, cerebral events such as intracranial hemorrhage, cardiac events or cardiac-related deaths and the progression, either symptomatic or asymptomatic, of the carotid artery disease were consider as future events.

*Methodology.* The proposed approach for the identification of individuals with the presence of Coronary Artery Disease (CAD) takes advantage of supervised learning, in which the model is trained based on known input and output data aiming to predict the future output given new input data. This section presents a supervised machine learning model that makes predictions, taking into consideration set of input data and known responses to the data output and trains a model to generate reasonable predictions for the response to new data. The overall pipeline of the proposed models overall training and evaluation procedure based on machine learning techniques comprises the following steps: 1. Data curation, 2. Problem formulation as ML, 3. Class imbalance problem handling, 4. Partition of the dataset into k-folds training and test set, 5. Data preprocessing, 6. Feature selection & classification scheme, 7. Model evaluation & performance metrics computation, and 8. Model selection.

*Problem Formulation as Machine Learning.* The developed machine learning models integrated into the first level of care of the risk stratification study has been formulated as a two-class classification problem, as it is described in the problem formulation section. This overall predictive supervised learning approach aims to learn a mapping from input features x to output Y, given a labeled set of input output pairs $D=\{(x_i,y_i)\}i=1N$, where D is the training set and N is the number of training examples. Each sample $(x_i,y_i)$ associates the input features with the carotid artery disease risk prediction Y, where $Y \in \{C_1,C_2\}$, is estimated by a non-linear parameterized function (f) of input features xERd, x= $[x_1,x_2,...,x_d]$. The goal of this supervised classification problem is to obtain an approximation F(x) of the function F $^*$(x) mapping the input x to output Y.

*Data Curation.* In this step, the data curation framework proposed by Pezoulas et al. (IEEE Open J. Eng. Med. Biol. 2020;1:83-90) is implemented in each utilized dataset and the framework outputs two different documents: (i) the data quality assessment report, and (ii) the curated dataset.

*Feature Selection and Evaluation Scheme Implementation.* As far as the classification procedure is concerned, input data are fed directly to classification algorithms. Specifically, the performance of six popular classification algorithms is examined to discriminate the high-risk group patients. The implemented algorithms are J48 algorithm, Random Forests (RF), Naive Bayes (NB), Support Vector Machine (SVM) and Artificial Neural Networks (ANN). In combination with the implementation of these classification schemes, we integrate in our methodology the implementation of feature selection techniques, to discard redundant features and maintain the more informative input subset, achieving in this manner high classifier performance. For this purpose, four different feature selection techniques are implemented, the Correlation based Feature Selection (CFS), the Class Attribute Evaluation (case 2), the Correlation Attribute Evaluation (case 3), the Gain Ratio (case 4), the InfoGain Ratio (case 5), the OneR (case 6), the principal components analysis (case 7) and the relief technique (case 8).

*Evaluation.* For evaluation purposes, the ten-fold cross validation is applied, which splits the initial subset into ten subsets, whereby the nine subsets are used for training and the remaining one subset is used for testing and after a full rotation, the results over the 10 testing sets are averaged in order to procure the overall performance of the algorithm. The feature selection techniques are repeated in every ten-fold repetition, ensuring that the feature selection procedure is based exclusively on the training dataset. The implemented classification schemes are evaluated using as metrics, the total accuracy of the model (the quotient of the correctly classified cases among the total cases), the area under curve (AUC) of each model, the sensitivity, specificity, the positive predictive value (PPV) and the negative predictive value (NPV).

*Validation of the models for carotid stenosis with retrospective dataset.* The validation was performed with 140 patients. Eighty-one patients and 59 patients were classified as Class 0 and Class 1, respectively, For each patient, the model provides a probability value, which corresponds to the probability of Class 1.Three probabilities for the cut-off threshold values were considered: Default cut-off 0.5 (Case 1), Cut-off 0.4 (Case 2) and Cut-off 0.56 (Case 3). Table 3 shows the results of this study.

Table 3. Validation results for the carotid stenosis model.

|  | Case 1 [Cut-off: 0.5] | Case 2 [Cut-off: 0.4] | Case 3 [Cut-off: 0.56] |
|---|---|---|---|
| **True Positive (TP)** | 39 | 46 | 36 |
| **True Negative (TN)** | 71 | 67 | 73 |
| **False Positive (FP)** | 10 | 14 | 8 |
| **False Negative (FN)** | 20 | 13 | 23 |
| **Accuracy** | 0.79 | 0.81 | 0.78 |
| **Sensitivity** | 0.661016949 | 0.779661 | 0.610169 |
| **Specificity** | 0.87654321 | 0.82716 | 0.901235 |
| **PPV** | 0.795918367 | 0.766667 | 0.818182 |
| **NPV** | 0.78021978 | 0.8375 | 0.760417 |

The results from the validation for the second problem, i.e., diagnosis of vulnerable plaque components, are shown in Table 4.

Table 4. Validation results for the diagnosis of vulnerable plaque components.

| | Diagnosis of vulnerable plaque components | | | | | |
|---|---|---|---|---|---|---|
| | Accuracy | Sensitivity | Specificity | PPV | NPV | AUC |
| **Subproblem 1 - Total Collagen** | 0.62 | 0.63 | 0.62 | 0.61 | 0.65 | 0.64 |
| **Subproblem 2 - SMCs Cells** | 0.64 | 0.54 | 0.74 | 0.41 | 0.83 | 0.68 |
| **Subproblem 3 - Lipid** | 0.63 | 0.66 | 0.6 | 0.59 | 0.68 | 0.65 |
| **Subproblem 4 - Neutrophils** | 0.58 | 0.49 | 0.68 | 0.55 | 0.63 | 0.64 |
| **Subproblem 5 - Macrophages** | 0.54 | 0.44 | 0.64 | 0.72 | 0.35 | 0.54 |

For the validation of the third problem, i.e., identification of SBLs, brain MRI analyses for 211 patients were annotated for the presence of infarcts (cortical, lacunar, active). In addition, the associate demographics, clinical, risk factors, serum markers with SBIs were statistically analyzed and the results are shown in Tables 5 and 6.

Table 5. Validation results for the stratification based on the presence of SBLs.

| | Class 0 (Absence of Cortical), N=165 | Class 0 (Presence of Cortical), N=46 | P value | Class 0 (Absence of Lacunar), N=203 | Class 0 (Presence of Lacunar), N=8 | P value |
|---|---|---|---|---|---|---|
| Height | 169.42 (8.85) | 171.16 (10.07) | 0.209 | 169.81 (9.197) | 170.57 (8.26) | 0.757 |
| Weight | 76.03 (12.08) | 77.47 (9.74) | 0.111 | 76.009 (11.49) | 85.14 (9.84) | 0.392 |
| BMI | 26.31 (3.37) | 25.96 (2.45) | 0.132 | 26.16 (3.16) | 28.37 (3.73) | 0.346 |
| SBP | 132.18 (16.71) | 128.16 (14.8) | 0.325 | 131.16 (16.54) | 134.75 (14.64) | 0.019 |
| DBP | 80.46 (8.09) | 77.81 (10.13) | 0.055 | 79.73 (8.66) | 81.50 (7.65) | 0.473 |
| Age | 70.25 (7.91) | 68.48 (6.43) | 0.242 | 69.82 (7.55) | 73.38 (8.62) | 0.552 |
| Cholesterol | 4.6 (1.08) | 4.62 (1.2) | 0.355 | 4.61 (1.1) | 4.15 (1.22) | 0.928 |
| LDL | 2.54 (0.99) | 2.63 (0.94) | 0.811 | 2.57 (0.99) | 2.18 (0.74) | 0.447 |
| HDL | 1.5 (0.56) | 1.42 (0.56) | 0.901 | 1.5 (0.57) | 1.22 (0.44) | 0.363 |
| Triglycerides | 1.52 (0.8) | 1.56 (0.83) | 0.726 | 1.49 (0.73) | 2.44 (1.75) | <0.001 |
| Glucose | 6.56 (2.35) | 6.03 (2.88) | 0.835 | 6.46 (2.5) | 6.12 (2.43) | 0.962 |
| | Class 0 (Absence of Small subcortical) N=185 | Class 0 (Presence of Small subcortical) N=26 | P value | Class 0 (Absence of Active) N=145 | Class 0 (Presence of Active), N=66 | P value |
| Height | 170.06 (8.83) | 168.74 (11.22) | 0.048 | 169.78 (8.64) | 169.93 (10.24) | 0.037 |
| Weight | 76.13 (11.55) | 78.09 (12.02) | 0.723 | 75.86 (11.88) | 77.42 (10.92) | 0.483 |
| BMI | 26.09 (3.17) | 27.07 (3.31) | 0.516 | 26.18 (3.28) | 26.36 (2.99)4 | 0.875 |
| SBP | 130.74 (16.28) | 135.64 (16.81) | 0.504 | 131.08 (16.29) | 131.81 (16.64) | 0.448 |
| DBP | 79.60 (8.55) | 81.44 (9.05) | 0.879 | 80.13 (8.08) | 79.34 (9.75) | 0.134 |
| Age | 69.92 (7.75) | 69.42 (7.03) | 0.517 | 70.12 (7.85) | 69.30 (7.16) | 0.585 |
| Cholesterol | 4.65 (1.11) | 4.29 (1.05) | 0.85 | 4.64 (1.08) | 4.53 (1.17) | 0.431 |
| LDL | 2.59 (0.99) | 2.3 (0.85) | 0.404 | 2.58 (1) | 2.51 (0.94) | 0.773 |

(continued)

| | Class 0 (Absence of Small subcortical) N=185 | Class 0 (Presence of Small subcortical) N=26 | P value | Class 0 (Absence of Active) N=145 | Class 0 (Presence of Active), N=66 | P value |
|---|---|---|---|---|---|---|
| HDL | 1.51 (0.57) | 1.34 (0.47) | 0.711 | 1.53 (0.57) | 1.38 (0.53) | 0.724 |
| Triglyceri des | 1.52 (0.81) | 1.59 (0.82) | 0.967 | 1.45 (0.72) | 1.71 (0.97) | 0.058 |
| Glucose | 6.42 (2.45) | 6.73 (2.73) | 0.383 | 6.5 (2.33) | 6.32 (2.79) | 0.518 |

Table 6. Validation results for the stratification based on the presence of SBLs (continued).

| | Presence of Cortical Infarct (N=46) | Odds Ration - CI | P value | Presence of Lacunar Infarct (N=8) | Odds Ration - CI | P value | Presence of Active Infarct (N=66) | Odds Ration -CI | P value |
|---|---|---|---|---|---|---|---|---|---|
| Sex (male), n(%) | 32 (24.4%) | 0.656 (0.326-1.323) | 0.237 | 8 (6.2%) | 0.938 (0.898-0.981) | 0.025 | 44 (33.6%) | 0.75 (0.407-1.381) | 0.355 |
| Sex (female), n(%) | 14 (17.5%) | | | 0 (0%) | | | 22 (27.5%) | | |
| Diabetes mellitus (no), n(%) | 36 (24.2%) | 0.604 (0.278-1.309) | 0.198 | 6 (4.1%) | 0.783 (0.154-3.992) | 0.768 | 49 (32.9%) | 0.771 (0.401-1.483) | 0.435 |
| Diabetes mellitus (yes), n(%) | 10 (16.1%) | | | 2 (3.2%) | | | 17 (27.4%) | | |
| Hypertension (no), n(%) | 7 (23.3%) | 0.909 (0.363-2.274) | 0.838 | 0 (0%) | 1.047 (1.014-1.080) | 0.246 | 6 (20%) | 2 (0.776-5.155) | 0.145 |
| Hypertension (yes), n(%) | 39 (21.7%) | | | 8 (4.5%) | | | 60 (33.3%) | | |
| Coronary disease (no), n(%) | 31 (20%) | | 0.298 | 5 (3.3%) | 1.973 (0.454-8.58) | 0.357 | 43 (27.7%) | 1.706 (0.867-3.358) | 0.12 |
| Coronary disease (yes), n(%) | 13 (27.1%) | | | 3 (6.3%) | | | 19 (39.6%) | | |
| Angina pectoris (no), n(%) | 26 (26.8%) | 0.372 (0.142-0.976) | 0.039 | 2 (2.1%) | 2.968 (0.479-18.378) | 0.222 | 33 (34%) | 0.681 (0.319-1.455) | 0.32 |
| Angina pectoris (yes), n(%) | 6 (12%) | | | 3 (6%) | | | 13 (26%) | | |
| Previous MI (no), n(%) | 36 (19.9%) | 2.685 (1.022-7.056) | 0.039 | 8 (4.5%) | 0.955 (0.926-0.986) | 0.335 | 52 (28.7%) | 2.481 (0.975-6.311) | 0.05 |
| Previous MI (yes), n(%) | 8 (40%) | | | 0 (0%) | | | 10 (50%) | | |
| Previous Cabg/pci (no), n(%) | 36 (20.9%) | 1.439 (0.589-3.516) | 0.423 | 6 (3.5%) | 2.025 (0.388-10.556) | 0.394 | 50 (29.1%) | 1.722 (0.767-3.868) | 0.184 |
| Previous Cabg/pci (yes), n(%) | 8 (27.6%) | | | 2 (6.9%) | | | 12 (41.4%) | | |
| Atherosclerosis of aortiliac segment or femoropopliteal crural (no), n(%) | 34 (23.4%) | 0.683 (0.303-1.543) | 0.358 | 6 (4.2%) | 0.913 (0.178-4.674) | 0.913 | 49 (33.8%) | 0.588 (0.283-1.221) | 0.152 |
| Atherosclerosis of aortiliac segment or femoropopliteal crural (yes), n(%) | 9 (17.3%) | | | 2 (3.8%) | | | 12 (23.1%) | | |

| | Presence of Cortical Infarct (N=46) | Odds Ration - CI | P value | Presence of Lacunar Infarct (N=8) | Odds Ration - CI | P value | Presence of Active Infarct (N=66) | Odds Ration -CI | P value |
|---|---|---|---|---|---|---|---|---|---|
| Aneurysm (no), n(%) | 40 (21.2%) | 1.242 (0.241-6.388) | 0.795 | 8 (4.3%) | 0.957 (0.929-0.987) | 0.55 | 58 (30.7%) | 0.753 (0.148-3.842) | 0.732 |
| Aneurysm (yes), n(%) | 2 (25%) | | | 0 (0%) | | | 2 (25%) | | |
| Statin (no), n(%) | 6 (31.6%) | 0.578 (0.207-1.616) | 0.381 | 1 (5.3%) | 0.696 (0.081-5.98) | 0.74 | 5 (26.3%) | 1.324 (0.456-3.843) | 0.605 |
| Statin (yes), n(%) | 40 (21.1%) | | | 7 (3.7%) | | | 61 (32.1%) | | |

**Blood Flow Model.** Blood flow modeling is used to generate a wide variety of hemodynamic parameters which, combined with US-derived data such as peak systolic velocity and stenosis degree for the analyzed artery, will act as input for the two models that generate risk scores either for plaque development and progression or for eventual cerebrovascular events. This is the key breakthrough of the combined blood flow model that brings it beyond the state of the art. The blood flow model comprises the first layer of the computational analysis of the 2nd and 3rd levels of the platform and has two main purposes: a) it provides essential data to the next layers of the computational process (i.e. Plaque growth module, Plaque rupture module), b) it provides a first layer of risk prediction for the development of atheroma and the possible occurrence of an event (i.e. stroke, ischemia etc.) and c) provides essential information for the ML models of the 1st level models of the platform. As it becomes evident, the final blood flow model is not a plain finite element blood flow simulation module that produces WSS, pressure and velocity values but a simple ML model that incorporates the aforementioned hemodynamic parameters calculated by the simulation and utilizes them to generate a risk regarding possible future cerebrovascular events (model 1) and a risk for development or progression of atheromatic lesions (model 2). The advantages of the blood flow model are the short period of time required to run the simulation and feed the model with the required simulation results, the fact that only the lumen geometry is required to perform the simulation and the excellent reconstruction quality of the arterial lumen, since only the TOF images are utilized. Furthermore, the accurate results of the simulation are of utmost importance since metrics such as TAWSS and arterial pressures are used for the more complex plaque growth model as inputs for the subsequent simulations.

*Datasets.* Blood flow modeling has been performed on a dataset of 60 baseline cases (87 arteries). Only baseline cases have been selected to be analyzed so far in order to train the model using the produced blood flow simulation results and the outcome of each case at follow-up 1 (FU1).

*Blood flow modeling.* Blood flow is generally modeled using the Navier-Stokes equations, as well as the continuity equation. The fluid density is $\rho$ and velocity components are $v_x$, $v_y$, $v_z$. The solution of the Navier-Stokes equations is achieved by using the finite element method. Blood was treated as a Newtonian fluid having a density of 1050 kg/m3 and a dynamic viscosity 0.0035 Pa · s.

*Boundary Conditions.* A patient-specific mass flow rate profile deriving from the US images is used for both the inlet (CCA proximal boundary) and the outlet of the ECA. At this stage, only the US approach is used to derive the respective flow rate profiles, since all cases provided with the required US images that include the flow velocity profiles for at least two of the three arterial branches. Briefly, each ultrasound image depicts the exact location of the velocity measurement along with the respective velocity waveform for several cardiac cycles. Specifically, when the velocity is measured at the common carotid artery, the measurement location is approximately 2 cm before the bifurcation site, whereas for the other two branches, the velocity is measured at the location of the maximum stenosis. If no stenosis is present, the measurement usually takes place right after the bifurcation site. In order to be accurate, the US images provide us with actual information on the exact location at which the velocity profiles are taken from. A post-processing step is then carried out, cropping the velocity diagram area from each US image and a dedicated MATLAB routine is used to capture and reproduce the diagram for an entire cardiac cycle. The patient-specific duration of the cardiac cycle cycle is then calculated using the pulse rate of the patient which is recorded in the electronic CRF repository. The diagram is then scaled according to the actual duration of the cardiac cycle and a dedicated script is then used to interpolate and extract that required velocity values for the time points that are required (i.e., time steps of 0.05 seconds). After the actual velocity values of each timestep are extracted, we then calculate the area at which the velocity profiles were measured at in order to calculate and produce the mass flow rate profile to be used for the transient simulation that is then carried out. The respective mass flow rate is calculated using the following equation:

$$m = \rho V A \quad (17)$$

where m is the mass flow rate, $\rho$ is the density of blood, V is the velocity of blood and A is the cross-sectional area of the velocity measurement location. The second important modification is the application of a zero-pressure boundary condition for the ICA boundary. The main reason for this is explained in the results section. Finally, a no-slip and no-penetration boundary condition is used for the arterial wall boundary, respectively.

*Mesh.* The lumen was discretized into tetrahedral elements, with a maximum element face size of 0.16 mm, with an increased mesh density throughout the boundary layer of the flow close to the arterial wall. The mesh size was defined after a detailed mesh sensitivity analysis (Table 7) performed on one right carotid artery model. The analysis was based on the correlation between the mesh size and the produced results regarding the average wall shear stress of a cross-sectional plane at the throat of the stenosis on 4 different mesh sizes. The location was specifically chosen due to its geometric and hemodynamic importance since it is a location at which the vulnerability of the plaque is at its peak. The mesh size with <5% difference in the generated WSS values was used in the final simulations. It should be noted that the computed pressure values at the outlet were also minimally influenced by the mesh size.

Table 7. Mesh sensitivity analysis. The row in bold indicates the selected final mesh.

| Face size (mm) | # of elements | Average cross-sectional WSS | % Error | Required computational time (minutes) |
|---|---|---|---|---|
| 0.12 | 1.16M | 14.221 | - | 4 |
| 0.14 | 875K | 14.459 | 1.68 | 3 |
| **0.16** | **680K** | **14.6564** | **3** | **2** |
| 0.18 | 540K | 15.12 | 6.3 | 1.5 |

*Results.* Blood flow simulations were carried out on a series of 87 carotid arteries deriving from 60 baseline cases in order to obtain all the necessary flow-related results that are used to train the blood flow risk model. a transient blood flow simulation is carried out for each case and the hemodynamic parameters calculated for each artery include TAWSS, intravascular pressure distribution, flow velocity, OSI, the ratio of areas of low TAWSS (i.e., <2 Pa) divided by the total vessel area and the ratio of areas of high OSI divided by the total vessel area, respectively. The first three parameters of interest are defined as shown below:

$$TAWSS = \frac{1}{T}\int_0^T |\tau_w| dt \quad (18)$$

$$OSI = \frac{1}{2}\left[1 - \frac{\left|\frac{1}{T}\int_0^T \tau_w dt\right|}{\frac{1}{T}\int_0^T |\tau_w| dt}\right] \quad (19)$$

$$RRT = \frac{1}{(1 - 2 \times OSI) \times TAWSS} \quad (20)$$

where T is a full cardiac cycle and $\tau_w$ is the instantaneous WSS. The OSI can be considered as the fraction of the angle and the magnitude change between the instantaneous wall shear stress and the time averaged wall shear stress. The values that can be assigned to it range between 0 and 0.5, where 0 depicts a fully unidirectional WSS whereas 0.5 is a value that represents an unsteady, oscillatory flow having a WSS of 0 Pa. In general, areas of high OSI values are prone to present with endothelial dysfunction and plaque development. Very low TAWSS values (i.e. <0.4 Pa) and high RRT values (>10 m2/N) are also known to be prone to atherogenesis, whereas high TAWSS values (i.e. >40 Pa) may create endothelial dysfunction and subsequent injury, thus increasing the risk of thrombosis. The pressure drop ratios between the CCA and the ICA or ECA outlets are calculated as follows: the pressure gradient throughout the entire vessel is calculated using a zero-pressure boundary condition for the ICA outlet. Having the patient-specific mean arterial pressure value for the CCA calculated using Eq. (21), the pressure difference for the simulated case between the CCA and the ECA is calculated and subtracted from the patient-specific value, thus calculating the actual pressure for the ICA outlet. The same procedure is then applied between the CCA and the ECA and the final ECA outlet pressure value is calculated, thus allowing for the calculation of the two pressure drop ratios, respectively.

$$MAP = \frac{SBP + 2(DBP)}{3} \quad (21)$$

Additionally, the ratios of areas of low TAWSS normalized by the total vessel area and the areas of high OSI normalized by the total vessel area, respectively, are calculated by creating two contours for each case that depict the areas of interest (area of TAWSS<2 Pa and area of OSI>0.4, respectively) and then calculating the areas of these contours.

*Validation.* The validation is performed and finalized by using 50% of the cases with occurring events as training dataset and the remaining 50% as validation dataset, respectively. The blood flow validation approach is based on the following assumption: using the US images which include the flow velocity profile, along with the artery of interest and the exact point at which the flow velocity is measured, this specific point on the MRI-based 3D arterial model is identified and a cross-section is created, at which the peak systolic velocity is calculated and compared to the US-based measurements.

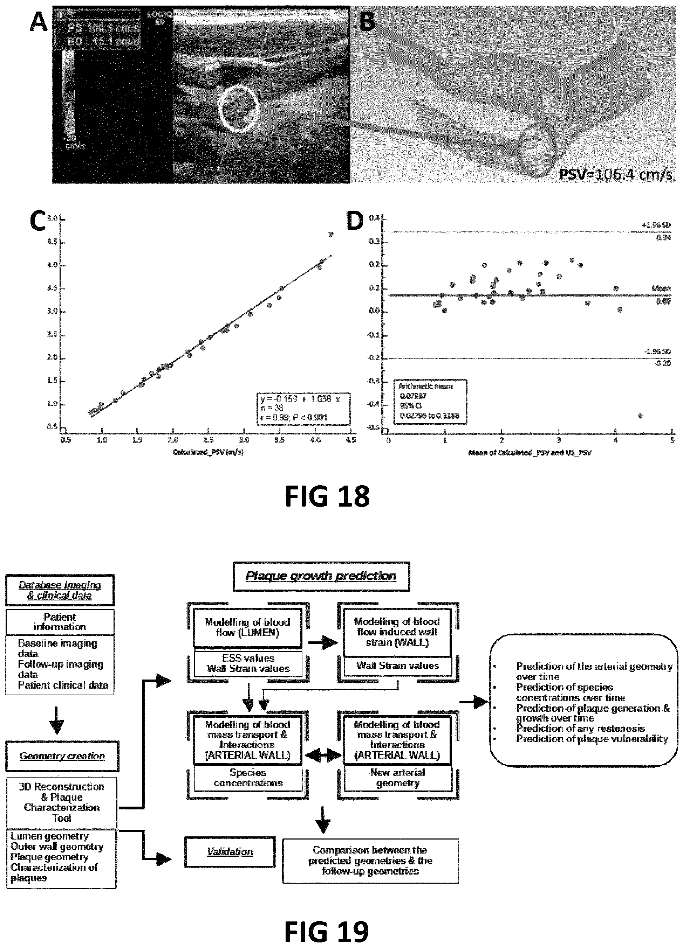

Figures 18A and B depict the whole blood flow validation process. The cross-section of interest at which peak systolic velocity is measured in the US sequences (yellow circle) is marked with a red circle on the right. The US-based PSV was 100.6 cm/s, whereas the calculated PSV from the blood flow simulation at the same spot was 106.4 cm/s, respectively. The calculated peak flow velocity values correlated well to the US-based measured ones, having a correlation coefficient of 0.99 (P<0.0001). Moreover, the two methods exhibited a good agreement with an average overestimation of the simulated flow velocities of 0.07337 m/s, having also 95% confidence intervals between -0.2758 to -0.1192 for the lower limit and 0.2659 to 0.4225 for the upper limit, respectively (P=0.0023). Figures 18C and D depict the regression plot and the Bland-Altman plot of the analysis, respectively.

*Plaque Progression Risk score - results and validation.* The formulation of the final plaque progression model involves treating the carotid artery progression as a sophisticated multivariate two-class classification problem, centering on the geometric findings of stenosis derived from the comprehensive US screening of each patient. In intricately crafting this model, a nuanced dataset analysis was conducted, categorizing arteries into two distinct classes. Class 0 encompasses arteries characterized by either no stenosis or minimal stenosis degree changes, while Class 1 comprises arteries demonstrating a stenosis degree change surpassing 10%. This formulation was guided by the discernment of medical experts, who identified a 10% increase in stenosis degree as a crucial threshold. The rationale behind choosing the 10% threshold is grounded in the need for clinical significance. Percentages below 10%, such as 5%, might not carry statistical significance and could potentially be attributed to imaging artifacts or even miscalculation arising from the minute differences in luminal diameter.

In the intricate classification process, the input data undergo direct input into five distinct classification algorithms, each tasked with discerning cases where the luminal diameter exhibits a propensity to narrow by more than 10%. The effectiveness of six renowned classification algorithms in fulfilling this critical task was explored. The selected classification algorithms encompass AdaBoost, Random Forest, Neural Network, Gradient Boosting, and Logistic Regression. To rigorously assess the prognostic model, a five-fold cross-validation process was employed, systematically dividing the initial input dataset into five subsets. Four subsets were allocated for training the algorithm, while the remaining one served as the test set. This cross-validation cycle was repeated, and the results from the five test sets were averaged to calculate the algorithm's overall performance. The objective was to ensure robust evaluation and validation of the predictive capabilities of each classification algorithm. In summary, the Neural Network classification scheme emerged as the top performer, achieving a notable 68% accuracy and a commendable 0.71 Area Under the Curve (AUC). The Random Forest scheme closely trailed behind, presenting compelling values with a 0.66 AUC and 64% accuracy. These outcomes underscore the potential of these algorithms in effectively discerning cases of significant luminal narrowing, offering valuable insights for clinical decision-making in the context of carotid artery health assessment. Furthermore, increased stenosis degree by more than 10% (i.e., Class 1) is correlated with higher Gini decrease values with Area of low TAWSS/Total vessel area, Peak TAWSS, PECA/PCCA, Area of high OSI/Total Vessel area (%) and ECA stenosis (%).

**Plaque Growth Model.** The Plaque Growth Model can predict real patient carotid atherosclerotic progress, with the potential to identify areas of risk. This helps predict new sites of plaque formation and evolution and ultimately provides input to the plaque rupture module to predict potential sites of plaque rupture. With plaque size becoming larger and lumen stenosis increasing, high WSS values are observed at the top of the plaque and low WSS values at the shoulders of the plaque. Disturbed flow and low WSS regions at the distal shoulder further increase endothelial dysfunction and plaque growth while supraphysiological high WSS levels at the top of the plaque have been associated with plaque destabilization and IPH. Both supraphysiological high WSS and increased tensile or PSS due to destabilized plaque components have been linked with the site of plaque rupture. In addition, different plaque burden metrics (plaque size parameters such as volume, cross-sectional area, thickness) and the presence or size of specific plaque tissue components (LRNC, IPH, thin or ruptured FC) has been associated with prior as well as future and recurrent events, including stroke. Carotid arterial WSS and PSS levels can be estimated in real patients via 3D reconstruction of their arteries and atherosclerotic tissue components from imaging (e.g., MRI), and subsequent performance of FEM analysis, whereas plaque burden metrics and plaque tissue components and size, can be measured by the automated plaque characterization tools disclosed herein.

*Classification of atherosclerotic lesions for plaque growth modeling.* The classification of atherosclerotic plaque lesions is performed according to the American Heart Association (Pelisek et al., J Clin Med. 2019;8(2). Types IV-VII are considered the most important and clinically relevant types of lesions. Concerning plaque stability/vulnerability, a thin fibrous cap $\leq$200 $\mu$m over a larger necrotic core is usually taken as a threshold indicating an unstable plaque (cap) prone to rupture caused by biomechanical stresses. Unstable/vulnerable plaques can develop from each plaque type V-VII. An ideal computational model of carotid atherosclerotic plaque growth and rupture should be able to simulate the main biological and biomechanical processes involved and include the types of clinically relevant atherosclerotic lesions (including tissue components) that have been associated with future cerebrovascular events. In addition, in order for a model to increase its clinical applicability it should enable its implementation in a patient-specific setting. However, a balance among the processes modeled in one hand and the computational resources and time required in the other hand should be reached, in order to maximize its clinical impact.

*Methodology.* The proposed methodology exploits real patient carotid artery geometries (e.g from MRI, CT, US) and personalized parameters. The model utilized as a first step the concentrations of LDL, HDL, and monocytes that enter the arterial wall in a WSS-dependent manner based on Kedem-Katchalsky Equations (plasma flow, LDL, HDL) and experimental data (monocyte transendothelial migration). Within the arterial wall, the main mechanisms of plaque formation are modelled based on modified Navier-Stokes Equations (plasma flow) and modified Convection-Diffusion-Reaction Equations. Carotid artery disease involves the deposition and accumulation of lipid species and leucocytes from blood into the arterial wall. Mass transport is influenced by blood flow in the vessel lumen and the transmural flow in the arterial wall. For lipoproteins such as LDL and HDL, the flux is based mainly on convective transport rather than diffusive transport. Blood velocity, derived from the Navier-Stokes equations solution, plays the most significant role in the mass transfer. Once the solution to these equations is obtained, the next step is the solution of the advection-diffusion equation. Concerning the boundary conditions of the problem, a concentration profile is applied at the inlet and outlet boundary. Suitable boundary conditions are also imposed at the wall.

The overall information flow of the developed plaque growth model is presented in Figure 19. Briefly, the patient MRI data are 3D reconstructed using the disclosed 3D reconstruction tool to obtain the domains of lumen, arterial wall and plaques. The reconstructed geometries along with the clinical data of each patient are used as input to the novel plaque growth model, which is performed in a 4-step procedure. The first step includes a steady-state simulation of the blood flow resulting to the evaluation of the WSS distribution of the endothelium. Following, the resulted flow-induced endothelial pressure is used as an initial condition to a static structural analysis of the arterial deformation to evaluate the arterial wall strain distribution. Both distributions of WSS and strain are used as input to the next step, which is the simulation of the mass transport and the interactions within the arterial wall. During this step, several equations are solved that account for the species dynamics, resulting to the evaluation of the plaque-induced strain distribution within the arterial wall. Finally, a static structural analysis of the arterial deformation is performed using the strain distribution as an initial condition, which provides the final wall thickening and lumen stenosis.

*1st step - Blood flow Model.* Initially, a steady state simulation of the luminal blood flow is performed using the Navier-Stokes equations considering a laminar, incompressible and Newtonian flow. In the overall analysis, a time duration of several months is examined to assess plaque evolution, and therefore a time step as small as a heart cycle cannot be applied that would include the pulsatile flow. To address this issue, the transient term of the Navier-Stokes equations is neglected, while the average patient-specific blood velocity is applied as a boundary condition to the blood flow model. The dynamic viscosity has been experimentally correlated to the patient-specific hematocrit, the concentration of total proteins minus albumin (TPMA) and the effective average shear rate (gamma).

*Boundary conditions.* The blood flow in lumen is conditioned by equations (22)-(24), which account for the patient-specific blood velocity and blood pressure, and the impenetrability of the endothelium layer.

$$\overrightarrow{u_{inlet}} = patient\ specific\ (\text{m/s}) \quad (22)$$

$$P_{outlet} = patient\ specific\ (\text{Pa}), \quad (23)$$

$$\frac{\partial u}{\partial n}_{endothelium} = 0. \quad (24)$$

*2nd step - Flow-induced strain of the arterial wall.* The second step of the analysis considers the flow-induced strain to the arterial wall. To evaluate the arterial wall strain distribution a linear stress-strain equation is implemented.

*Boundary conditions.* A frictionless support is applied to the adventitia and the arterial wall side areas.

*3rd step - Atherosclerotic process.* The third step includes the modeling of the main mechanisms of plaque formation. Species transport is influenced by the transmural flow of plasma in the arterial wall, which is modeled by the modified Navier-Stokes that account for the decreased flow space and the momentum losses due to arterial wall's porosity. The momentum losses are due to the permeability decrease, according to Darcy's law, as well as due to the velocity direction changes, which are neglected in this case, because of the low plasma velocity within the arterial wall. In the domain of wall, all the considered species are described by the modified convection-diffusion-reaction considering the porosity effects. This equation derives from the mass conservation of the studied substance, and includes a transient term, an advection term, a diffusion term and a source or sink term. The blood concentration of the substances is patient-specific and depends on several endogenous and exogenous factors. However, they can also infiltrate into the arterial wall, participating in the atherosclerotic process. Within the wall, their dynamics is described by the convection-diffusion-reaction equation, which is modified to account for flow in porous media. Within the arterial wall, an inflammatory response initiates as LDL molecules are oxidized by reactive oxygen species (ROS), produced initially by dysfunctional endothelial cells in areas of disturbed flow and low WSS. HDL levels mitigate this process by being competitively oxidized, causing the decrease of ROS. The correlation between HDL concentration and HDL oxidation rate was defined experimentally in (Kontush A., Curr Opin Lipidol. 2010;21(4):312-8). An equal value to the LDL oxidation rate is also assumed for HDL. Oxidized LDL causes the production of inflammatory mediators attracting monocytes that differentiate into macrophages to uptake the oxidized LDL to form foam cells, the cornerstone of plaque tissue formation. The advection term of all cells is neglected due to their large size, which move exclusively due to diffusion forces. Cytokines cause, among other, the differentiation of the contractile SMCs into synthetic SMCs. Their production is enabled after the inflammatory signaling of both the OxLDL and the macrophages. Cytokines are assumed to remain in the membrane of the macrophages and thus their motion is disregarded. The differentiated synthetic SMCs produce collagen promoting the generation of a fibrotic tissue that ultimately transforms atheromatus plaque to fibroatheromatic tissue, enabling the simulation of the fibrous cap formation surrounding the LRNC of the plaque. In the arterial wall, naïve CD4+ and CD8+ T cells are activated by IL12. In turn, the activated CD4+ T cells produce IL2 and IFN-γ. The kinetics of naive CD4+ and CD8+ T cells, active CD4+ and CD8+ T cells, IL2, IL12 and TGFβ are also described by Convection-Diffusion-Reaction equations.

The activated CD8+ T cells attack and kill SMCs within the inflamed fibroatheromatic tissue, simulating in this way the process of the fibrous cap thinning and destabilization of the plaque tissue. Cytotoxic CD8+ T cells are increased more than 3-fold in unstable versus stable human carotid plaques. Atherosclerotic plaques having a thin fibrous cap become unstable, and more vulnerable to rupture under the biomechanical forces (i.e. high PSS) applied by the pulsatile blood flow and may result ultimately in thromboembolism manifested as stroke. Apoptotic bodies from SMCs have been shown experimentally to calcify within 21 days following their formation, a period used to simulate the generation of calcified atherosclerotic plaque tissue from apoptotic SMCs. Elegant studies have revealed an inverse relationship between microcalcification area and collagen content within the fibrous cap of human carotid plaques, while microcalcifications are predicted to produce significant fibrous cap stress mainly localized in regions of low collagen content. Similar findings today indicate that atherosclerotic plaques with dense calcifications (macrocalcification) are more likely to represent stable plaques, while small, spotty, or fragmented calcified regions (microcalcifications) are associated with early-stage plaques or unstable lesions prone to rupture or erosion.

$$\gamma \frac{\partial c_{Calcification}}{\partial t} = k_{calcification} c_{Apoptotic\ SMCs} \quad (25)$$

where, $k_{calcification}$ is the calcification rate of apoptotic SMCs. TGF-β is mainly produced by SMCs and macrophages, it is localized in the shoulder region and the periphery of the lipid core and it is more abundant in the asymptomatic than in the symptomatic plaques. TGF-β signaling in T-cells increases the inflammatory cell content and activation in atherosclerotic lesions and decreases SMC and collagen content consistent with a more vulnerable and unstable plaque phenotype.

$$\gamma \frac{\partial c_{TGFB}}{\partial t} = -k_{d_{TGFB}} c_{TGFB} + \text{F8}(WSS) c_{Synthetic\_SMCs} \quad (26)$$

where, $k_{d\_TGFB}$ is the degradation rate and F8(WSS) is an experimentally defined equation that describes the production rate of TGFB in regard to WSS values.

$$\text{F8}(WSS) = 4.61 \times 10^{-4} \text{ WSS [pg h}^{-1}] + 6.15 \times 10^{-5} [\text{pg h}^{-1}] \quad (27)$$

Finally, it is known that TGF-β levels counter-regulate the activation of naïve CD4+ T cells by promoting their differentiation to Foxp3+ T-regulatory cells (Tregs). This key anti-inflammatory T-cell population has been identified in the fibrous cap, shoulders and lipid core regions of human carotid endarterectomy lesions presenting a 3.5 fold decrease in unstable compared to stable plaques. The suppressive capacity of Tregs is simulated by the inhibition of the CD8+ and CD4+ T cell proliferation which has been shown experimentally to be dependent by the Treg/T cell target ratio.

$$\gamma \frac{\partial c_{Treg}}{\partial t} + \frac{\partial}{\partial x_i}\left( (\boldsymbol{K} \cdot \boldsymbol{U})_j c_{Treg} \right)$$
$$= D_{Tcells} \frac{\partial}{\partial x_i}\left( \boldsymbol{K} \cdot \frac{\partial}{\partial x_j} c_{Treg} \right) - k_{d\_Treg} c_{Treg}$$
$$+ c_{N_{CD4}} \text{F4}(c_{TGFB}) \text{F5}(c_{IL2}) \quad (28)$$

where, $k_{d\_Treg}$ is the degradation rate of Tregs. Foam cells (lipid core), synthetic SMCs with collagen (fibrotic tissue) and calcified apoptotic bodies (calcified tissue) comprise the atherosclerotic plaque volume while the size and location of each plaque tissue component together with the changing hemodynamic environment determine the plaque vulnerability to rupture under the biomechanical stresses applied by the pulsatile blood flow.

$$Ratio_{\frac{Plaque\ volume}{Finite\ Volume}}$$
$$= c_{Foam\ cells} * F\_volume + c_{Synthetic\ SMCs} * S\_volume$$
$$+ c_{collagen} * G\_volume \tag{29}$$

where *F_volume, S_volume* and *G_volume* are the volumes of one foam cell, one syvthetic SMC cell and the specific volume of collagen, respectively. The volume of Foam cells is calculated by their average radius. The SMCs are cylindrical cells with an average and therefore their volume is calculated using their average radius and their average length. The average specific volume of collagen is the inverse of its density.

*Boundary Conditions - Endothelial Flow rates.* The endothelial fluxes are defined by the Kedem-Katchalsky equations, which evaluate the fluxes through bio-membranes, based on the pressure and concentration differences across them. However, despite the fluxes of LDL and HDL, which are well described by the Kedem-Katchalsky equations, the endothelial monocyte and T-cell fluxes cannot be described by them. Specifically, the monocyte and T cell transendothelial migration is based both on the inflammatory signaling, which is more intense in case of a high OxLDL concentration, and on the magnitude of the endothelial shear stresses, which do not allow easy cell infiltration within the arterial wall in areas with high WSSs.

$$J_{monocytes} = \frac{m_r}{1 + (WSS/WSS_0)} c_{OxLDL} c_{monocytes,\ lumen}, \tag{30}$$

$$where,\ C_{monocytes,\ lumen} = 5,3\% C_{Leukocytes,\ lumen},$$

$$J_{Tcells} = TEM_{Tcells} C_{Tcells,\ lumen} \tag{31}$$

$$where,\ TEM_{Tcells} = \begin{cases} \frac{1}{25}(-9.93WSS^3 + 11WSS^2 - 4,1WSS + 0.55);\ 0 \le WSS \le 0.41Pa \\ 0;\ WSS > 0.41Pa \end{cases} \tag{32}$$

$$and\ \ C_{Tcells,\ lumen} = 30\% C_{Leukocytes,\ lumen}. \tag{33}$$

where *Lp* is the hydraulic conductivity of the endothelium, which depends on the endothelial wall shear stresses. *DPLDL* and *DPHDL* are the diffusive permeabilities of the LDL and HDL respectively, which depend on the endothelium's nitric oxide concentration. The above equations imply that both monocytes and T-cells concentration values are correlated to the individual patient specific value of total white blood cells obtained from the eCRF of the prospective study

$$L_P = (0.2077x10^{-12} ln(WSS + 0.015) + 3.1588x10^{-12}) \tag{34}$$

$$DP_{LDL} = DP_{HDL} = (-6\ c_{NO} + 0.34). \tag{35}$$

where, WSS is the wall shear stress value to blood flow. Nitric oxide is the regulator of the arterial contraction and dilation, and it is produced by the endothelial nitric oxide synthases (eNOS). Nitric oxide concentration has been correlated with the partial pressure of oxygen and the eNOS concentration.

$$c_{NO} = c_{NO,\ max} \left( \frac{p_{O_2}}{p_{O_2} + k_M} \right) \tag{36}$$

$$c_{NO,max} = 1.26\ c_{eNOS}. \tag{37}$$

The concentration of eNOS has been experimentally correlated to the magnitude of the endothelial wall shear stresses.

$$c_{eNOS} = (0.0033\ ln(WSS) + 0.0322) \tag{38}$$

The boundary conditions of the rest species to endothelial layer are defined below.

$$\frac{\partial c_{OxLDL}}{\partial n} = \frac{\partial c_{Macrophages}}{\partial n} = \frac{\partial c_{T-reg}}{\partial n} = \frac{\partial c_{TGFB}}{\partial n} = \frac{\partial c_{IFNG}}{\partial n} = \frac{\partial c_{IL2}}{\partial n} = \frac{\partial c_{IL12}}{\partial n}$$
$$= \frac{\partial c_{cytokines}}{\partial n} = \frac{\partial c_{Foam\ cells}}{\partial n} = \frac{\partial c_{Contractile\ SMCs}}{\partial n} = \frac{\partial c_{Synthetic\ SMCs}}{\partial n}$$
$$= \frac{\partial c_{collagen}}{\partial n}$$
$$= \frac{\partial c_{A\_CD4}}{\partial n} = \frac{\partial c_{A\_CD8}}{\partial n} = 0, \qquad (39)$$

*Boundary Conditions - Adventitia.* The following boundary conditions are applied to the adventitia layer.

$$P_{plasma} = P_{adventitia} \qquad (40)$$

$$c_{LDL} = kA \cdot c_{LDL,\ lumen} \qquad (41)$$

$$c_{HDL} = k_A \cdot c_{HDL,\ lumen} \qquad (42)$$

$$\frac{\partial c_{OxLDL}}{\partial n} = \frac{\partial c_{Macrophages}}{\partial n} = \frac{\partial c_{T-reg}}{\partial n} = \frac{\partial c_{TGFB}}{\partial n} = \frac{\partial c_{IFNG}}{\partial n} = \frac{\partial c_{PDGF}}{\partial n}$$
$$= \frac{\partial c_{IL2}}{\partial n} = \frac{\partial c_{IL12}}{\partial n}$$
$$= \frac{\partial c_{cytokines}}{\partial n} = \frac{\partial c_{Foam\ cells}}{\partial n} = \frac{\partial c_{Contractile\ SMCs}}{\partial n} = \frac{\partial c_{Synthetic\ SMCs}}{\partial n}$$
$$= \frac{\partial c_{collagen}}{\partial n}$$
$$= \frac{\partial c_{A\_CD4}}{\partial n} = \frac{\partial c_{A\_CD8}}{\partial n} = \frac{\partial c_{N\_CD4}}{\partial n} = \frac{\partial c_{N\_CD8}}{\partial n} = 0, \qquad (43)$$

*Boundary Conditions - Wall Side Areas.* The following boundary conditions are applied to the wall side areas.

$$U_{plasma} \cdot \vec{n} = 0 \cdot \vec{n} \qquad (44)$$

$$c_{LDL} = k_A \cdot c_{LDL,\ lumen} \qquad (45)$$

*4th step - Arterial wall thickening & lumen narrowing.* In the last step, the arterial wall thickening and lumen narrowing is evaluated using a linear stress-strain equation. The rationale is that the simulated plaque volume causes the thickening of the arterial wall, and therefore causes the development of volumetric strains, which can be used by the stress-strain equation to predict the wall's thickening.

$$Ratio_{\frac{Plaque\ volume}{Finite\ Volume}} = c_F * F_{volume} + c_S * S_{volume} + c_G * G_{volume} + c_T * T_{volume} = \frac{\partial V}{V}, \qquad (46)$$

$$\underbrace{\frac{\partial V}{V} = \frac{(l+dl)^3 - l^3}{l^3} \qquad\qquad \varepsilon = \frac{dl}{l}}, \qquad (47)$$

$$\frac{\partial V}{V} = (1 + \varepsilon_x)(1 + \varepsilon_y)(1 + \varepsilon_z) - 1 \Rightarrow \frac{\partial V}{V} \approx \varepsilon_x + \varepsilon_y + \varepsilon_z \qquad (48)$$

However, in this approach, the arterial wall thickening is constrained to occur mainly in the direction of the centerline, and therefore the volumetric strain results to the Eq. (49)

$$\frac{\partial V}{V} = \frac{(l + dl)l^2 - l^3}{l^3} = \frac{dl}{l} = \varepsilon_{directional} \qquad (49)$$

*Boundary Conditions.* A frictionless support is applied to the adventitia and the arterial wall side areas.

$$\frac{\partial \varepsilon}{\partial n}_{adventitia} = 0 \quad (50)$$

$$\frac{\partial \varepsilon}{\partial n}_{arterial\ wall\ side\ areas} = 0 \qquad (51)$$

Using this approach, wall thickening is enabled by simulating the inward remodeling of the vascular wall (wall thickening). Initial plaque tissue components identified by cardiovascular MR imaging (i.e. LRNC, calcified and fibrotic tissue) at baseline, are included in the model as impermeable structures, while the quiescent SMCs of the wall are assumed as fibrotic tissue.

*Modeling of hypertensive conditions.* The mathematical model utilizes the hypertension-related variables summarized in Table 8 and have been used by Sakellarios et al. (Am. J. Physiol. Heart Circ. Physiol. 2013; 304(11):H1455-1470) in early mathematical models of plaque growth, showing the differences of LDL accumulation in normal and a high blood pressure.

Table 8. Hypertension-related variables.

| | Normal blood pressure | Hypertension |
|---|---|---|
| Adventitia Pressure | 17.5 mmHg | 30.5 mmHg |
| Adventitia LDL concentration | $0.005c_{LDL,lumen}$ | $0.015c_{LDL,lumen}$ |

*Prediction of plaque volumes of different types.* Recently, Pleouras et al. presented the first valid method to predict and generate plaque geometries after a certain period of time, using their model results (Annu Int Conf IEEE Eng Med Biol Soc. 2020:2020:2808-2811). To achieve that, a 3-step procedure was implemented. The first step concerns the plaque growth simulation in a patient dataset, that resulted in the evaluation of the species' concentrations within the arterial wall, as well as the wall thickening over time. The second step includes the registration between the follow-up and the baseline geometries, which was performed manually. The third step includes the statistical correlation of the follow-up plaque regions with the model's predicted plaque component concentrations. This resulted in the evaluation of specific minimum thresholds for the simulated plaque component concentrations, which are used as conditions to characterize plaque regions (lipid, calcified).

*Numerical Implementation.* The plaque growth model was employed using the ANSYS Inc. Software. In particular, the ANSYS Mesh module was implemented for the discretization of the geometries, the ANSYS CFX module was used for the fluid dynamics part of the model and the ANSYS Structural module was used for the simulation of wall thickening and lumen narrowing. The presented mathematical model is implemented in a fourstep process in Ansys. For each one, the same discretized geometry was implemented. Specifically, tetrahedral elements of 0.2mm edges were used to discretize lumen, wall and any available plaques. This value resulted from a sensitivity analysis that was performed to validate the mesh quality. Moreover, several other techniques were used to improve the quality of the mesh, such as the patch-independent technique and inflation.

Besides mesh specification, one more analysis was performed to specify the time step of the transient simulation of the atherosclerotic process. Initially, a simulation was performed with a time step of 3 months that provided the maximum plasma velocity. Subsequently, a valid assumption of the maximum time step was evaluated by implementing the Courant number equation. Specifically, the Courant number is a convergence-indicator in transient simulations, linking time step, maximum plasma velocity and the average element length, and it was developed by Courant, Friedrichs and Lewy (https://en.wikipedia.org/wiki/Courant%E2%80%93Friedrichs %E2%80%93Lewy_condition). Accordingly, the resulted timestep of 0.5 day was tested, by evaluating the results of three different simulations of a patient's atherosclerotic process, which had a time step of 0.2-, 0.5- and 1-day periods. Finally, all simulations presented minor differences, and therefore the time-step of 0.5 day was selected for the implementation of this model to the available patients.

*Results.* Using the above-mentioned methodology, the novel plaque growth model was implemented in eleven 3D reconstructed carotid arterial segments of 7 patients. Patients that underwent surgery are not suitable for the model's validation, because the change of their carotid segment geometries does not result from the atherosclerotic process, but from the professionals' artificial configuration. Most patients presented a slight stenosis progression to at least one of the main carotid branches. For each patient, the simulation time period resulted from the interscan period between the baseline and the follow-up examinations. In each case a time step of 0.5 days was applied, as it derived from the corresponding analysis. Additionally, several patient-specific clinical values were also used as input to the model, including Mass Flow Rate (RCCA, LCCA), Systolic Blood Pressure, Diastolic Blood Pressure, Hematocrit, Blood LDL, Blood HDL, Blood Glucose.

In the steady state simulation of the blood flow within the 3D reconstructed lumen, which is the model's first step, several flow-related variables are predicted. Among the results, the distribution of the endothelial shear stress is necessary for executing the model's following steps. Figures 20 A, B illustrate a case example of a 3D reconstructed carotid segment along with the simulated streamlines, as well as the derived endothelial shear stress distribution. Figure 20A depicts case examples of the streamlines of a 3D reconstructed carotid bifurcation, which are colored according to the magnitude of velocity, and Figure 20B depicts the endothelial shear stress distribution. During the second step of the analysis, the necessary arterial wall strains are predicted, which is achieved by implementing a one-way interaction approach. Figures 20 C, D show the predicted arterial wall strain distribution of the same 3D reconstructed carotid segment, as in Figures 20 A, B, and specifically the endothelial strain distribution (C) and the arterial wall strain distribution (D). The model's third step includes the transient simulation of all species' dynamics within the arterial wall. Initially, in this step, a steady state analysis is performed to evaluate the accurate plasma dynamics within the arterial wall, considering the effects of arterial wall's porosity, as well as the peculiarities of the endothelial layer (Figure 20E). Following, using the resulted plasma flow within the arterial wall as the transient model's initialization, the species dynamics are predicted using the convection-diffusion-reaction equations. The concentration of each substance is evaluated in every time step, while transient results are kept in a frequency of 120 hours, to lower disk space occupation. It was observed that in the region that follows lumen stenosis in the right branch, there is an increased concentration of the related to atherosclerosis substances. This complies with the fact that the progression of stenosis is usually happening towards the direction of the blood flow. Using the resulted species concentration and the available thresholds for the simulated plaque component concentrations, plaque regions are predicted (Figure 20 F, G) and specifically the predicted lipid (F) and fibrous (G) plaque regions of a 3D reconstructed carotid bifurcation. Finally, during the model's last step, the carotid wall thickening and lumen stenosis are evaluated using the derived plaque strain as input to a structural analysis. An indicative result is shown in Figures 20 H-J, depicting the progression of lumen stenosis & wall thickening over 1 year of a high-risk patient's 3D reconstructed carotid bifurcation, where (H) corresponds to the initial arterial geometry, (I) 0.5 year later and (J) 1 year later. Thus, going beyond the state-of-the-art of the plaque growth model results, the inventors are introducing the prediction of several flow-related and atherosclerosis-related metrics, such as localization of future plaque regions, stenosis progression, lesion length progression, prediction of peak wall shear stress and pressure drop in the future lumen geometries.

*Validation.* A comparison of the simulated and the actual follow-up carotid arteries at the first year follow-up visit is performed using the validation strategy presented by Stone et al. (Circulation. 2012;126:172-181). The rationale of the selected validation approach is to evaluate the mathematical model's capability to predict the evolution of the plaques in the baseline carotid arterial segments (CCA, ICA, ECA) to match corresponding segments in the subsequent follow-ups. To achieve this, 0.5 mm-distanced cross-sections from each segment (both at baseline and follow-up) is used as a means of validation. To eliminate possible registration errors, six sequential cross-sections were combined for all arterial geometries (baseline, simulated and follow-up) and their mean average area was evaluated, and used as a means of validation. The evaluation of the mean average values of each available variable was performed for the CCA, ICA and ECA. The preliminary results of the validation process, which was applied to 11 different carotid bifurcations, indicated the model's accuracy to predict the stenosis progression, as well as the generation of new stenotic regions. The comparison of the simulated and the real follow-up carotid lumen areas resulted to a significant statistical correlation (r=0.671, P<0.0001).

**Agent Based Model.** The problem of atherosclerosis includes components that contribute to plaque formation and progression, each with proper characteristics, behaviors, and sets of rules. The interaction between these components and the environment they develop determines plaque progression. For this reason, the inventors chose the Agent Based Model (ABM) as the right approach to reproduce the evolution of plaque progression and arterial reshaping by simulating the behavior of agents. The key components of agent-based models are agents, rules, environment and timescale. ABM provides a way to capture the complexity and heterogeneity of atherosclerosis by representing individual components and their interactions. This approach allows researchers to explore emergent phenomena, understand system dynamics, and test hypotheses in a simulated environment.

*Methodology.* The methodology followed is based on Corti et al. (Comput. Biol. Med. 118, 103623) that consists of four iterated steps: 1) preparation of the geometry, 2) CFD simulation, 3) ABM simulation, and 4) generation of the new 3D geometry. During the step of geometry preparation, 3D model of healthy artery is built and a 3D mesh of the fluid domain is generated using PAK software. In order to compute the hemodynamics and extract the WSS values at the lumen interface of 2D vessel cross-sections, CFD simulation is performed in PAK software. For each cross-section, the hemodynamic-driven remodeling is repeated with implemented ABM that simulates the cellular, extracellular and lipid dynamics. The WSS values are calculated with CFD simulation, while the ABM has been used for the arterial wall remodeling. In each 2D cross-section, all geometry changes from the ABM were transformed to fluid domain which cause blood flow calculation and again WSS determination for next step to update the ABM. Each geometrical change computed by the ABM in 2D cross-sections affects the fluid dynamics domain. For that reason, the ABM module is coupled to the CFD module and by this coupling, the WSS distribution is always updated. The 2D ABM replicates remodeling of the arterial wall by simulating cell mitosis, production or degradation of the ECM and lipid infiltration in the intima. Also, different vessel structures and compositions were implemented, as well as dealing with new cellular events.

The ABM behavior was validated in an environment with atherogenic conditions. CFD-ABM coupling was performed to initialize the ABM with hemodynamic input. 2D circular cross-section composed of 3 concentric layers (tunica intima, media and adventitia) made initial position. The 2D ABM is fed with an initial WSS profile Bentzon et al., Circ Res. 114(12), 1852-1866). Also, low WSS affects endothelial function by reducing atheroprotective genes and increasing the atherogenic ones, which leads to atherosclerotic plaque formation.

$WSS_c$ values were obtained from the 3D CFD simulation. A level of endothelial dysfunction $D^i$ was computed as given in Eq. (122). $WSS^i$ is the WSS at site $i$ and $WS$ $S_o = 1Pa$ is the WSS threshold.

$$D(WSS)^i = D^i = \begin{cases} 1 - \dfrac{WSS^i}{WSS_0}, & if\ WSS^i < WSS_o \\ 0, & otherwise \end{cases} \qquad (52)$$

$WSS_c$ was set following the study by Samady et al. (Circulation. 2011;124(7):779-788). Each dysfunctional endothelial site $i$, with $D^i \neq 0$, starting a state of alteration that diffuses within the intima through isotropic diffusion, from a peak of intensity $D^i$ with a diffusion constant $\varphi$. $A^{i,k}(D^i, d)$ is the level of alteration produced by the $i$-th endothelial site and recorded at the $k$-th site within intima, at a distance d from $i$ (Eq. (53)).

$$A^{i,k}\left(D^i, d\right) = A^{i,k} = D^i * e^{-\frac{1}{2}\left(\frac{d}{4\varphi t}\right)^2}. \qquad (53)$$

For each site k, the individual states of alteration are summed up to define the global level of inflammation of the k-th site $I^k$ in Eq. (124). $N_L$ is the initial number of sites of the lumen wall and the resulting $I^k$ affects the agent dynamics.

$$I^k = \sum_{i=1}^{N_L} A^{i,k}. \qquad (54)$$

If all the WSS values at the $i$-th sites are larger than the threshold, $D^i = 0 \forall i$ and $I^k = 0$ everywhere. If $WSS^i < WSS_0$, a state of inflammation $I$ develops and the mechanisms of plaque formation are activated. Then, related WSS profile was defined as atherogenic. The baseline densities of probability were set for cell mitosis/apoptosis and ECM deposition/degradation to replicate the physiologic conditions. They were defined with Eq. (55) and (56), respectively.

$$p_{mit} = p_{apop} = \alpha_1, \qquad (55)$$

$$p_{prod} = \beta * p_{deg} = \alpha_4 \qquad (56)$$

where $\alpha_1, \alpha_4$ and $\beta$ were involved in order to maintain the physiological cell/ECM ratio which is defined for each tissue layer during the phase of initialization.

Based on Garbey et al. (J. Theor. Biol. 2017;429:149-163), coefficient $\beta$ for the intima, media and adventitia layers were set in order to guarantee stable trends of ECM in each layer under baseline conditions. With the specified calibrated coefficients, Eq. (125) and Eq. (126) trigger physiological remodeling of the wall, leading to the replication of the homeostatic state of a healthy artery. The probability of cell mitosis and ECM production in the intima increases with the inflammation level which directly cause the number of neighbouring lipids and the closeness to the lumen, leading to the following:

$$p_{mit} = \begin{cases} \alpha_1 \cdot \left(1 + \alpha_2 I^k\right) if\ n_{lip} = 0 \\ \alpha_1 \cdot \left(1 + \alpha_2 I^k\right)\left(1 + \alpha_3 n_{lip}\right)\left\{1 + \exp\left(-d_{lumen}^k\right)\right\} if\ n_{lip} \neq 0 \end{cases} \qquad (57)$$

$$p_{prod} = \begin{cases} \alpha_4 \cdot \left(1 + \alpha_2 I^k\right) \ if \ n_{lip} = 0 \\ \alpha_4 \cdot \left(1 + \alpha_2 I^k\right)\left(1 + \alpha_3 n_{lip}\right)\left\{1 + \exp\left(-d_{lumen}^k\right)\right\} \ if \ n_{lip} \neq 0 \end{cases} \qquad (58)$$

where $\alpha_2$ and $\alpha_3$ weight the effect of the inflammation state $I^k$ and the influence of the neighboring lipids $I^k$ while $d_{lumen}^k$ is the distance between the site $k$ and the lumen wall. The coefficients were set following the framework proposed by Corti et al. Lipid dynamics is activated once the intima thickens over a given threshold. The probability of lipid infiltration is computed as the probability of a site k expressed by:

$$p_{lipid} = \alpha_5\left(1 + I^k\right)\left\{1 + \alpha_6 \cdot \exp\left(-d_{lip}^k\right)\right\}\left(1 + \frac{n_{lip}}{\alpha_7}\right), \quad (59)$$

where $\alpha_5$ sets the event probability in the interval (0, 1). The terms $\alpha_6 \cdot \exp\left(-d_{lip}^k\right)$ and $\left(1 + \frac{n_{lip}}{\alpha_7}\right)$, promote lipid clustering, by increasing the probability of a lipid to occupy a site $k$ close to another lipid, whose distance is $d_{lip}^k$. At each time step only one lipid can enter the intima. The terms and coefficients of Eq. (59) are set so to obtain a lipid nucleus resembling histological characteristics. When lipids enter the intima layer, the lipid agents must maintain their position throughout the entire simulation. Therefore, the agent movement is performed along the shortest path that does not include the lipid agents. This allows maintenance of the lipid core. Movement of all agents must always comply with the minimum energy principle, except in the mentioned case when the lipid agents are positioned along the shortest path.

A unique 3D geometry of the lumen vessel is built and starting ABM configuration of each plane for the following cycle is determined. Specifically, for each ABM solution of a given cross-section M, lumen and external radius, as well as plaque thickness, were computed and indicated as $R_j^i(\vartheta)$, with $j$=1,2,3, respectively.

The corresponding deviation, $\Delta^i$, from the average configuration, $\overline{R_j(\vartheta)}$ was computed as defined in Eq. (60), and the ABM $i$-th output minimizing $\Delta$ was selected:

$$\Delta^i = \sum_{j=1}^{3} \int_0^{2\pi} w_j \sqrt{\left(R_j^i(\vartheta) - \overline{R_j(\vartheta)}\right)^2} d\vartheta, \quad (60)$$

where each $j$-th quantity is weighed by $w_j$. The same criterion was applied for all cross-sections and the 3D geometry was finally reconstructed.

*Coupling FE computational fluid dynamics with ABM.* Blood flow dynamics can be successfully modeled using continuum methods such as the FEM. By numerically solving various forms of Navier-Stokes equations it is possible to obtain velocity and pressure fields, as well as distribution of shear stresses along the blood vessel wall. Recent research has shown that hemodynamic parameters can have significant influence on atherosclerosis development process. WSS has a significant influence on LDL transport from blood-to-blood vessel wall, therefore has also influence on speed of atherosclerosis development. On the other hand, atherosclerosis development process is a consequence of intensive interactions at a molecular level inside the vessel wall. The whole process involves several types of cells and molecules that behave according to specific rules. The process is induced by LDL molecules that are transferred into the blood vessel wall. This clearly indicates that molecular processes of atherosclerosis are dependent on hemodynamic parameters of blood flow. Having in mind the dependency between the macro process of blood flow and the micro process of atherosclerosis development, a coupled model of the finite element model and agent-based model was developed. Blood flow through a straight blood vessel with stenosis was modeled using the finite element method. Fluid dynamics computation is performed using PAK-F finite element solver (Kojic M et al., 1998; PAK-F Finite Element Program for Laminar Flow of Incompressible Fluid and Heat Transfer. Kragujevac, Serbia), giving velocity and pressure field, as well as WSS distribution. Flux of LDL entering the vessel wall is calculated by solving Kedem-Katchalsky equation. The process of atherosclerosis development inside blood vessel wall is modeled using ABM, where an unfolded wall cylinder is taken as the domain of interest. The whole domain area is divided into a grid of cells (spots) where agents interact. The size of spots is chosen so that each spot can accept only one macrophage, one of the key actors in atherosclerosis process. The agent-based model parameters are taken from literature. The rate of LDL molecule entering the domain of interest is not constant, and they are taken from the results obtained using the finite element model. Axial LDL flux distribution along the blood vessel is mapped onto the horizontal axis of the agent-based model. Source spots of the LDL inflow into the agent-based model are uniformly distributed along the horizontal axis, with the entering rate in each LDL source spot is proportional to the LDL flux at the corresponding axial coordinate of the FE model. The distribution of the LDL source spots along the vertical axis is random. Using such boundary conditions, simulation of atherosclerosis development by agent-based method is performed.

*Example: Straight blood vessel with stenosis - 2D axisymmetric - Finite Element Model.* Blood flow through a straight vessel with stenosis is modeled using the finite element method. The axisymmetric model of vessel consists of 9331 nodes and 9000 2D-axisymmetric elements. Blood velocity at the vessel entrance is set according to physiological conditions. Concentration of LDL in blood is set to unity. As a result of the FE calculations, axial distribution of LDL flux from blood to wall is obtained.

*Agent-based model.* The agent-based model corresponds to the wall segment in unfolded state and has dimensions 4x2 mm. Taking into account the macrophage size, spots were chosen to be of 20x20 $\mu$m, so that the entire grid consists of 200x100 cells. As a boundary condition, the rate of LDL inflow at each LDL source spot is prescribed to be proportional to the flux of LDL at the corresponding axial position of the FE model.

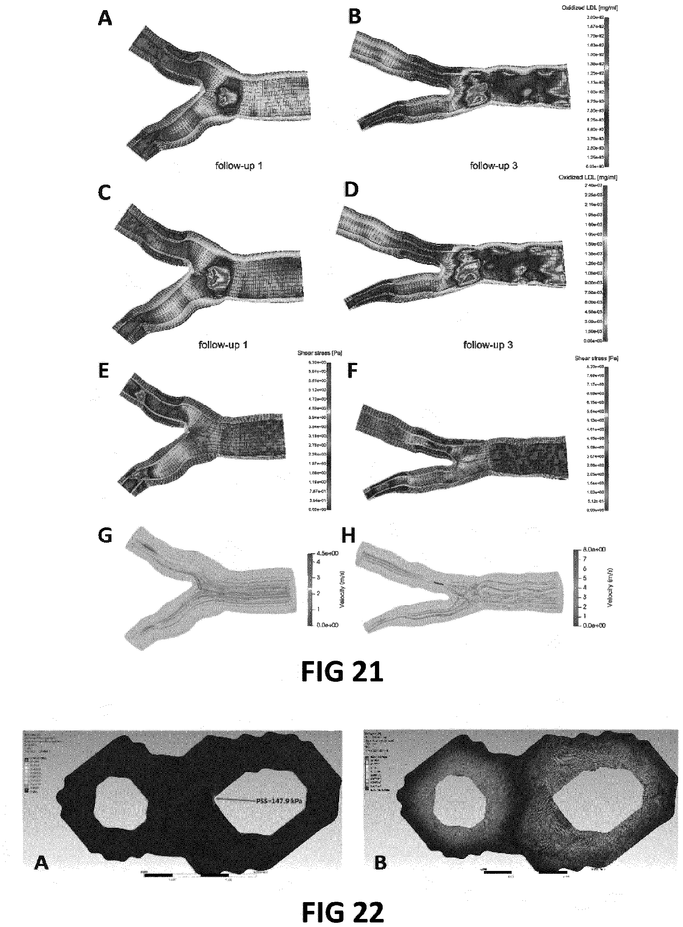

*Results and validation in 3D.* The atherosclerotic progression within the 3D models of carotid arteries was simulated using PAK Athero (Filipovic N et al., PAK Athero, Finite element program for atherosclerosis. 2022; BIOIRC doo Kragujevac, Serbia) and PAK ABM approach. The results are presented at the follow up 1 and follow up 3 in order to compare disease progression. The fluid and solid domains were separated. Also, wall domain included separated plaque area. In wall domain the ABM was used as 8 agents inside each finite element. The results for plaque progression for two different time periods (follow up 1 and follow up 3) with the representation of different parameters for US-reconstructed models are presented in Figure 21. The Figure, showing results for a randomly chosen patient, presents oxidized LDL using PAK Athero (A, B) and PAK ABM (C, D), shear stress (E, F) and velocities (G, H) at follow up 1 (A, C, E, G) and follow up 3 (B, D, F, H). The ABM model was used with convergence 1.e-5 per time step. The time domain was simulated with increased flux for follow up 1 and follow up 3. The results for follow ups were compared with available clinical measurements which showed that oxidized LDL in PAK ABM model is a little higher then in PAK Athero model. That is probably because ABM functions (57) and (58) give higher values for the same shear stress and agent movements are stronger then continuum level of calculation which was implement in PAK Athero model. Lipid dynamics described in Eq. (59) is also faster than continuum approach which gives more realistic scenario of plaque growing due to interaction of the different agents moving and relation between them.

**Plaque Rupture Model.** Several patient-specific parameters such as arterial geometry, peak systolic blood velocity, pressure, plaque constituents and plaque morphology, affect the biomechanical stresses which are produced in the arterial wall. Both supraphysiological high WSS and increased PSS due to plaque components that are on the verge of destabilization have been associated with plaque rupture. In addition, diverse plaque burden metrics such as volume, cross-sectional area or thickness of the plaque components and the presence or size/volume of specific plaque tissue components has been linked with prior, future and recurrent symptoms or events, including stroke. The plaque rupture model that has been developed is a cumulative model which utilizes the calculated carotid WSS and PSS levels from the finite element analysis, as well as plaque burden metrics and plaque tissue components and size, which are measured by the automated plaque characterization tool.

*Methodology.* Biomechanical features including WSS distribution throughout the vessel (proximal-middle-distal maximum and minimum WSS) the pressure drop throughout both branches (i.e. ICA and ECA), the TAWSS, the OSI (i.e. >0.4) and the RRT are extracted from the Blood flow module described above. A significant feature that is also extracted and used for risk assessment and stratification is the PSS, which is then extracted using 2D structural analysis of the MRI slices of interest. In order to assess the risk of plaque rupture, the inventors initially used the established PSS thresholds from current literature, which was later adapted to the clinical study cohort when follow up visits were finished, and all cases presented with a cerebrovascular event were collected.

Regarding the 2D structural analysis, the process begins with the automated segmentation of the lumen, the outer wall and the plaque components that are derived from the plaque characterization module which is previously described. The 2D borders of the lumen, the outer wall, and the plaque (i.e., slices with a sufficient plaque area which results to a plaque burden of >40%) are then translated into a 2D model in cartesian coordinates, using the respective pixel spacing value that is assigned for each clinical center as it is stated within the respective DICOM tags. Due to its small distance from the luminal border (i.e., <150 $\mu$m), the area that is between the lipid portion and the lumen is assumed to be a thin fibrous cap. The 2D model is then discretized into finite elements using an element size of 0.07 mm for the arterial wall and an element size of 0.05 mm for the plaque constituents. The two element sizes have been chosen after a dedicated mesh sensitivity analysis that was conducted during the duration of the project. Moreover, an inflation approach is also followed creating 2 layers of brick elements at the interfaces between the luminal border and the arterial wall, as well as the plaque components and the arterial wall, respectively. The final 2D mesh of the indicated case consisted of 29000 elements.

Regarding the boundary conditions of the model, the outer edge of the outer wall is fixed with a frictionless support to prevent rigid body displacement. The plaque components are bonded to the arterial wall at all times and the luminal border is the interface at which the maximum site-specific pressure that is previously calculated during the blood flow simulation is applied as a loading condition. In order to compensate for the pressure drop along the stenosis region, we initially performed a simple blood flow simulation using patient-specific boundary conditions and then used the calculated pressure at the cross-section which corresponded to the segmented slice.

The next step of the modeling process includes that material properties assignment for the arterial wall and the plaque component that is involved in the examined slice. Hyperelastic material properties were assigned to the lipid and fibrous plaques and a linear elastic model for the arterial wall. Regarding the arterial wall, a Mooney-Rivlin approach was used, whose parameters were C10=0.07 MPa, C20=3.2 MPa, C21=0.0716 MPa and the remaining parameters were set as cij=0. The incompressibility parameter d of the arterial wall was d= $1.0 \times 10-5$ Pa-1. The material properties of the plaque components were derived from the stretch ratio to Cauchy stress diagrams that were created after a uniaxial extension study (Teng Z et al., Acta Biomater. 2014:10(12):5055-5063). The diagrams were used to calculate and extract the respective 5-parameter Mooney-Rivlin constants. *Results.* Each examined case constituted of one or more 2D slices with a calculated plaque burden of >40%. In this context, each case could include the analysis of as many as 4-5 slices per artery (i.e., left and right carotid). As expected, the highest PSS value that is calculated from each case with the 2D structural analysis is the one that is used for the plaque rupture model that incorporates several geometrical factors, biomechanical parameters and calculated hemodynamic factors in order to make the plaque rupture prediction. To this point, a set of 20 cases have been used to calculate the plaque structural stresses and use them as input for the plaque rupture model. Figure 22A depicts an indicative case (#12) from the clinical trial, where the calculated PSS value was 147.9 kPa, thus exceeding the threshold of 135 kPa that has been documented in the literature. Interestingly enough, this specific case was documented as one of the study's events (i.e., the specific subject died from stroke between BL and FU1 timepoints). Figure 22B depicts the total deformation for the same examined case.

Several prior art studies utilize the same approach regarding plaque vulnerability assessment. A 2D structural analysis on the 2D segmented contours (the vast majority using coronary vessels due to the flexibility provided by the VH-IVUS technique) is performed by applying patient-specific pressure boundary conditions on the interface between the lumen and the arterial wall. However, the patient-specific pressure value that is usually used is the mean aortic pressure that is applicable at the inlet of the common carotid artery. When a severe stenosis is present at any of the two main carotid branches (ICA or ECA), the peak pressure at the site of the throat of the stenosis is bound to present with a significantly lower value, a fact that has not been considered. The present invention deals with this inaccuracy by performing a 3D patient-specific blood flow simulation (using the rigid-wall assumption) and calculating the pressure distribution within the carotid vasculature. This is done using the initial blood flow model which is the first model of levels 2 and 3 of the risk stratification tool. This way, the actual pressure value is extracted at the site that corresponds to the segmented slice of interest and then this value is applied as a correct loading condition for the 2D analysis to follow. The calculated PSS values are incorporated to the plaque rupture model, which, along with the other parameters that are taken into account, produces a plaque rupture risk score. This aspect is missing from current literature and similar platforms and is considered as one of the main features that are beyond the state of the art.

The disclosed methodology involves utilizing the initial blood flow model, the foundational model of levels 2 and 3 in this risk stratification tool. Through this comprehensive simulation, the precise pressure value is extracted at the location corresponding to the segmented slice of interest. This extracted value is then employed as an accurate loading condition for subsequent 2D analyses. This meticulous process ensures that the 2D analysis captures the actual pressure conditions at the site of stenosis, rectifying the inaccuracies prevalent in other studies. The calculated PSS values are seamlessly integrated into the plaque rupture model. This incorporation, along with other pertinent parameters, contributes to the generation of a comprehensive plaque rupture risk score. Notably, this approach distinguishes the present invention from the current literature and not only rectifies a critical oversight in existing studies by accounting for pressure variations in stenotic regions but also presents an integrated and comprehensive model for assessing plaque rupture risk.

**Risk Stratification Tool.** *Architecture - Modular Design:* A) AI Non-imaging Risk Assessment Module: Leveraging machine learning techniques, this module processes clinical and demographic data to make early predictions about potential risks. It provides an initial assessment even before the processing of any imaging data. B) 3D Arterial Reconstruction: This central module interacts with sub-modules dedicated to MRI, US, and CT inputs, reconstructing precise 3D models of the carotid arteries. Advanced algorithms process image slices, stitch them together, and project an accurate representation of the patient's arterial structure. C) Blood Flow Simulation: Once the 3D model is in place, this module simulates blood flow through the arteries, providing insights into areas of potential concern due to altered hemodynamics. D) Plaque Rupture & Progression Simulation Modules: These predictive tools utilize patient-specific data and broader clinical knowledge to forecast potential plaque rupture events and the progression of the plaque over time.

Each module functions as a microservice. This means that every module operates independently, allowing for focused updates, troubleshooting, and scaling. The microservices communicate through well-defined interfaces, ensuring seamless integration and data flow between them. To ensure global accessibility and real-time data processing, the RISK Stratification Tool employs cloud integration. All microservices are cloud-native, designed to exploit the elasticity of cloud resources. APIs ensure secure and seamless data transfer, both within the system and when interfacing with external tools or databases.

Databases: a) Non-imaging Data Database: A dedicated database holds non-imaging clinical data, ensuring quick retrieval during the AI risk assessment phase. b) ORTHANC DB for Medical Images: Recognizing the unique requirements of storing and accessing medical images, the ORTHANC database has been employed. It is optimized for DICOM image storage, allowing for efficient image retrieval and processing.

*Flow of clinician - user interface (UI) interaction.* A medical professional interacts with the UI to input patient data. The UI sends a request via the API Gateway. Depending on the request, the API Gateway routes it to the respective microservice, which processes the data. Data may be fetched from or stored into the appropriate database during this process. Once processing is complete, results are returned to the user through the UI.

*Implementation in the Risk Stratification Tool - API Gateway.* This is the central point through which all requests pass. It routes each request to the appropriate microservice, ensuring efficient and streamlined operations.

- Dedicated Databases: Each microservice has access to a dedicated database (either the general DB for non-imaging data or ORTHANC DB for medical images) that fits its unique data needs, ensuring speed and efficiency.

- Inter-service Communication: While each microservice operates independently, there are instances where they need to communicate. This is achieved through well-defined APIs, ensuring that data exchange between services is seamless and efficient.

- Service Orchestration: While each service operates autonomously, there are processes in place to ensure that they operate harmoniously, with clear orchestration mechanisms ensuring that processes run in correct sequence.

Adopting a microservices architecture for the Risk Stratification Tool ensures it is robust, flexible, and futureproof. By decoupling the components, it can quickly adapt to advancements in medical science and technology, ensuring it remains a cutting-edge tool in the stratification of carotid artery disease patients.

*Risk assessment module.* The Key Components of this module are: 1) Data Collection Interface: Enables the input of raw data, ensuring all necessary fields are populated with accurate and relevant information. 2) Preprocessing Unit: Cleanses, standardizes, and normalizes the data, making it suitable for machine learning algorithms. This involves handling missing values, encoding categorical variables, and scaling numerical data. 3) Machine Learning Models: A suite of algorithms trained on vast datasets to make predictive determinations. These models might include decision trees, neural networks, and ensemble methods, which are chosen based on their performance metrics. 4) Model Selection & Tuning: Dynamically chooses the most appropriate model based on the type and nature of input data. Uses cross-validation to tune hyperparameters and optimize model performance. 5) Feature Importance Extractor: Identifies which input variables (features) are most influential in the risk determination, aiding clinicians in understanding the primary risk factors for a particular patient.

The Functionalities of the Risk assessment module involve: 1) Data Ingestion: Collects and structures non-imaging data from diverse sources. 2) Predictive Modeling: Deploys the most appropriate ML model to analyze the input data and determine the risk profile. This might involve classification (high risk, medium risk, low risk) or regression (predicting the probability of an event). 3) Interpretable AI: Uses techniques like SHAP (SHapley Additive exPlanations) or LIME (Local Interpretable Model-agnostic Explanations) to explain model decisions in human-readable terms, making the AI's decisions transparent and justifiable. 4) Results Presentation: Converts the raw output of the machine learning models into a format that's readily understandable by clinicians. Includes visual representations like risk scores, charts, and graphs that highlight the primary concerns and areas of interest.

*3D geometry reconstruction module.* This advanced module is tasked with generating accurate three-dimensional reconstructions of the carotid arteries, using imaging data from MRI, US, and CT scans. By visualizing the vessel's structure in high-definition 3D, clinicians can more effectively detect, monitor, and assess plaque buildup and other anomalies. The key components are: 1) Imaging Data Importer: Accepts and processes raw imaging data from MRI, US, and CT scans. Ensures compatibility and corrects for any discrepancies or inconsistencies between different imaging sources. 2) Preprocessing Engine: Removes noise and enhances contrast in the input imaging data. It utilizes advanced algorithms to distinguish between the arterial structure and surrounding tissue. 3) 3D Reconstruction Algorithms: A set of sophisticated algorithms that combine the cross-sectional images from the scans to generate an interactive 3D model of the artery. 4) Visualization Renderer: This provides an interface for clinicians to interact with the 3D model, allowing for zoom, rotation, and exploration of the arterial structure in detail. 4) Annotation & Marking Tool: Allows clinicians to mark areas of interest, such as regions of significant plaque accumulation, directly on the 3D model.

*Blood flow simulation module.* The primary goal of this module is to recreate the blood flow dynamics within the 3D model of the carotid arteries. It offers a dynamic visualization and analysis of how blood moves, interacts with plaque formations, and might be impeded by potential obstructions. This real-time simulation assists clinicians in assessing the severity and potential risks associated with specific arterial conditions. The key components are: 1) Flow Dynamics Engine: Utilizes CFD principles to model and visualize blood flow patterns, including velocity, turbulence, and pressure gradients. 2) Input Data Integrator: Accepts the 3D reconstructed geometry of the arteries and other patient-specific data like blood viscosity and heart rate. 3) Boundary Condition Setter: Allows the setting of inlet and outlet conditions, such as flow rate and pressure, replicating patient-specific circulatory conditions. 4) Simulation Renderer: Provides real-time, high-definition visualizations of the blood flow, highlighting areas of slow flow, turbulence, or potential stenosis. 5) Analysis Toolkit: Assists clinicians in evaluating the simulation data, extracting vital metrics like shear stress, pressure drop, and recirculation zones. *Plaque growth module.* The Plaque Growth Module aims to predict and visualize the growth trajectory of arterial plaques over time. By simulating the progression of plaque buildup, this module helps clinicians anticipate potential complications and adjust treatment strategies proactively. The key components are: 1) Plaque Analysis Engine: Extracts and characterizes existing plaque features from the 3D arterial model, assessing factors like size, location, and composition. 2) Growth Prediction Algorithm: Incorporates patient-specific data and factors (like cholesterol levels, blood pressure, and genetic predispositions) to estimate plaque growth rates and patterns. 3) Visual Representation Tools: Offers a detailed graphical visualization of plaque progression over specified time intervals, aiding in intuitive understanding and monitoring. 4) Patient History Integrator: Takes into account past medical records, lifestyle habits, and medication intake to refine predictions.

*Database Structures.* A) Relational Databases (RDBMS) utilize tables to store data and establish relationships between them. It is perfect for structured data, such as patient profiles, clinical records, and metadata. It supports data integrity, ensures consistency, and offers complex query capabilities. B) Orthanc Database is a lightweight, yet powerful, DICOM server for healthcare imaging. It manages and stores medical images like MRI, CT, and US. It provides a RESTful API, ensuring easy integration with the platform's microservices, and supports DICOM web standards for seamless image exchange. C) NoSQL Databases offer flexible data models and is scalable, ideal for heterogeneous data. They are optimal for omics data (transcriptomics, miRNAomics, proteomics, lipidomics), as this data doesn't fit into the structured paradigm of traditional relational databases. The basic features are Horizontal scalability, schema flexibility, and high performance for large volumes of data.

*Model Categorization.* To effectively apply the ASME V&V 40 framework, it is essential to categorize the computational models within the Risk Stratification Tool. This categorization helps in focusing the verification and validation efforts and aligning them with the specific functionalities of each model. Table 9 is a representation of the model categorization.

Table 9. Model categorization within the Risk Stratification Tool.

| Model Category | Description | Primary Function | Data Types Used | Intended Use |
|---|---|---|---|---|
| **AI Non-Imaging Risk Assessment** | ML algorithms to assess risk | Analyze clinical data to predict disease risk | Clinical, demographic, genetic data | Preliminary risk stratification |
| **3D Arterial Reconstruction** | Computational algorithms for 3D modeling | Convert 2D imaging (MRI. US, CT) into 3D models | MRI, Ultrasound, CT scan data | Visualizing arterial structure and plaque formation |
| **Blood Flow Simulation** | Fluid dynamics models for blood flow analysis | Simulate blood flow through 3D arterial models | Hemodynamic data, 3D model parameters | Assessing blood flow patterns and potential obstructions |
| **Plaque Rupture Prediction** | Algorithms for assessing plaque vulnerability | Predict potential sites of plaque rupture | Data from blood flow simulations and 3D models | Identifying high-risk areas for intervention |
| **Plaque Progression Simulation** | Predictive models for plaque growth | Project the progression of arterial plaques over time | Historical imaging data, patient demographics | Anticipating future plaque development |

**Claims**

1. A computer implemented method for the risk stratification of asymptomatic patients with carotid artery disease, the method comprising:

a) storing, using a database, medical data including non-image medical data and carotid imaging medical data for a plurality of patients, wherein said non-image medical data comprise demographics, clinical characteristics, data from blood examinations and medication intake;

b) inputting one or more non-image medical data values into a plurality of algorithms to produce a first set of outputs;

c) inputting one or more carotid imaging data values into a plurality of algorithms comprising at least a plaque evolution prediction model for predicting plaque progression, stabilization, or regression; and/or a plaque rupture prediction model utilizing plaque burden metrics such as volume, cross-sectional area and/or thickness of the plaque components, plaque composition metrics and/or biomechanical features; to produce a second set of outputs that provide plaque evolution and risk prediction for plaque rupture; and

d) combining the first set of outputs with the second set of outputs to provide an overall risk estimation for plaque progression, plaque rupture, cerebral and cardiovascular disease outcomes for said asymptomatic patients.

2. The method according to claim 1, wherein step (c) comprises the following sub-steps:

c1) inputting one or more carotid imaging data values from ultrasound, magnetic resonance imaging and/or computed tomography into a plurality of texture-based image processing algorithms and machine learning models in a hybrid iterative framework, said algorithms comprising at least a 3D carotid reconstruction algorithm and a plaque characterization algorithm, to produce as output a 3D arterial geometry model and a plaque tissue geometry and composition model, respectively,
wherein said 3D arterial geometry model comprises a 3D reconstructed lumen model, a 3D outer wall model and a 3D plaque constituents model;

c2) inputting the 3D reconstructed lumen model, imaging data and electronic case report form (eCRF)-derived features such as stenosis degree and hypertension, into a blood flow modeling algorithm based on hemodynamic features calculation and machine learning, to produce as output the risk for plaque progression;

c3) inputting the 3D reconstructed lumen model, the 3D outer wall model, the 3D plaque constituents model and imaging-derived features such as stenosis degree and/or hypertension, into a cerebrovascular event prediction model based on hemodynamic features calculation, plaque structural stress calculation and machine learning, to produce as output a cerebrovascular event risk score;

c4) inputting the output of steps (c2) and (c3) into a Finite Element Method (FEM)-based plaque growth algorithm to provide as output the concentrations of plaque agents comprising LDL, HDL, OxLDL, OxHDL, monocytes, microphages, Foam Cells, T-Cells, Cytokines, smooth muscle cells, Collagen and/or plaque volume,

c5) repeating steps (c1) to (c4) at different time points, thereby providing prediction of plaque evolution over time.

3. The method according to claim 2, wherein the ultrasound images comprise patient scans encompassing the common carotid artery, its branches, and the carotid bifurcation, captured from transversal and longitudinal perspectives; and wherein said images are annotated with captured plaque components in order to produce the dataset inputted into the plaque characterization algorithm of step (c1).

4. The method according to anyone of the preceding claims, wherein the algorithms that produce the first set of outputs comprise machine learning models for the diagnosis of patients having a degree of stenosis above 50% in at least one of their carotid arteries; diagnosis of vulnerable plaque components such as Lipid Rich Necrotic Core or Intraplaque Hemorrhage; evolution of silent brain lesions; evolution of brain structural changes; and/or prognosis of cerebral outcomes or events such as stoke, transient ischemic attack, atrial fibrillation or silent brain lesions.

5. The method according to claim 4, wherein step (b) further comprises the substep of estimating the accuracy, sensitivity, specificity, positive predictive value, negative predictive value and/or area under the curve of the machine learning models; and wherein the outputs produced by said plaque vulnerability prediction models comprise histology-based features comprising the concentration of total collagen, smooth muscle cells, red oil, neutrophils and/or macrophages.

6. The method according to anyone of the preceding claims, for the identification of asymptomatic carotid stenosis patients to whom to recommend ultrasound screening, for the identification of asymptomatic patients with higher risk for stroke to whom to target surgical intervention such as CEA or carotid stenting (CAS) versus best medication therapy, and/or for providing advice on the optimal time for the next follow-up screening of asymptomatic patients who are not eligible for surgical intervention.

7. The method according to anyone of the preceding claims, wherein said circulating biomarkers comprise LDL, HDL, OxLDL, OxHDL, extracellular vesicle (EV) proteins, ceramides, EV ceramides and/or serum proteins.

8. An apparatus for the risk stratification of asymptomatic patients with carotid stenosis, comprising:

a database configured to store medical data including non-image medical data and carotid imaging medical data for a plurality of patients;
a digital processor configured to execute the instructions to perform a method according to any one of claims 1 to 7; and
a user interface configured to provide the generated risk stratification output/risk score to the patient and/or a physician.

9. A non-transitory computer readable medium for use on a computer system containing computerexecutable programming instructions configured, when executed by a processor of the computer system, to perform a method according to any one of claims 1 to 7.

**FIG 1**

FIG 2

FIG 3

**FIG 4**

**FIG 5**

Lumen area
(annotated)

Hausdorff distance

Lumen area
(estimated)

# FIG 6

TN

FN

FP

TP

# FIG 7

**FIG 8**

FIG 9

**FIG 10**

**FIG 11**

**FIG 12**

**FIG 13**

**FIG 14**

**FIG 15**

FIG 16

FIG 17

**FIG 18**

**FIG 19**

**FIG 20**

**FIG 21**

**FIG 22**

## FIG 23

Europäisches
Patentamt

European
Patent Office

Office européen
des brevets

## EUROPEAN SEARCH REPORT

**Application Number**

EP 23 22 0881

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | TSAKANIKAS VASSILIS D ET AL: "TAXINOMISIS: A cloud - based platform for risk profiling and patient specific management of the carotid artery disease", 2023 45TH ANNUAL INTERNATIONAL CONFERENCE OF THE IEEE ENGINEERING IN MEDICINE & BIOLOGY SOCIETY (EMBC), IEEE, 24 July 2023 (2023-07-24), pages 1-4, XP034487899, DOI: 10.1109/EMBC40787.2023.10340947 [retrieved on 2023-12-11] | 1,6-9 | INV. G16H50/20 G16H50/30 G16H50/50 G16H30/40 |
| Y | * p. 1, left column, par. 2 p. 1, right column, par. 3 p. 2, left column, par. 1 p. 2, left column, par. 2 p. 2, left column, par. 3 p. 2, Table 1 par. spanning p. 2 and p. 3 p. 3, left column, last par. p. 3, right column, par. 1 p. 3, right column, par. 2 p. 3, Figure 1 and description thereof * ----- | 2-5 | |
| Y | MANTZARIS MICHALIS ET AL: "A Multimodal Advanced Approach for the Stratification of Carotid Artery Disease", 2019 IEEE 19TH INTERNATIONAL CONFERENCE ON BIOINFORMATICS AND BIOENGINEERING (BIBE), IEEE, 28 October 2019 (2019-10-28), pages 706-709, XP033680509, DOI: 10.1109/BIBE.2019.00133 [retrieved on 2019-12-24] * p. 707, right column, last par. p. 708, left column, par. 2 * ----- -/-- | 2,3,5 | |

TECHNICAL FIELDS
SEARCHED (IPC)

G16H

The present search report has been drawn up for all claims

| Place of search | Date of completion of the search | Examiner |
|---|---|---|
| Munich | 27 May 2024 | Mühlbauer, Max |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 23 22 0881

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | PLEOURAS DIMITRIOS S ET AL: "Prediction of the atherosclerotic plaque development in carotid arteries; the effect of T-cells", 2022 44TH ANNUAL INTERNATIONAL CONFERENCE OF THE IEEE ENGINEERING IN MEDICINE & BIOLOGY SOCIETY (EMBC), IEEE, 11 July 2022 (2022-07-11), pages 1590-1593, XP034182014, DOI: 10.1109/EMBC48229.2022.9871632 [retrieved on 2022-09-08] * p. 1591, section "C. Plaque Growth Model" * | 5 | |
| Y | KIGKA VASSILIKI I ET AL: "Detection of Asymptomatic Carotid Artery Stenosis through Machine Learning", 2022 44TH ANNUAL INTERNATIONAL CONFERENCE OF THE IEEE ENGINEERING IN MEDICINE & BIOLOGY SOCIETY (EMBC), IEEE, 11 July 2022 (2022-07-11), pages 1041-1044, XP034183143, DOI: 10.1109/EMBC48229.2022.9870927 [retrieved on 2022-09-08] * par. spanning p. 1041 and p. 1042 p. 1044, left column, Table * | 4,5 | |

-----

-----

-/--

TECHNICAL FIELDS
SEARCHED       (IPC)

The present search report has been drawn up for all claims

| Place of search | Date of completion of the search | Examiner |
|---|---|---|
| Munich | 27 May 2024 | Mühlbauer, Max |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

**Application Number**

EP 23 22 0881

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | Branko Arsic: "Atherosclerotic Plaque Characterization Using Deep Learning Methods", AORTA, 10 June 2022 (2022-06-10), pages 1-2, XP93167203, DOI: 10.1055/s-0042-1750994 (http://dx.doi.org/10.1055/s-0042-1750994 Retrieved from the Internet: URL:https://www.thieme-connect.de/products/ejournals/abstract/10.1055/s-0042-1750994 [retrieved on 2024-05-27] * the whole document * ----- | 1-9 | |
| A | TSAKANIKAS VASSILIS D ET AL: "A deep learning oriented method for automated 3D reconstruction of carotid arterial trees from MR imaging", 2020 42ND ANNUAL INTERNATIONAL CONFERENCE OF THE IEEE ENGINEERING IN MEDICINE & BIOLOGY SOCIETY (EMBC), IEEE, 20 July 2020 (2020-07-20), pages 2408-2411, XP033816515, DOI: 10.1109/EMBC44109.2020.9176532 [retrieved on 2020-08-24] * the whole document * ----- | 1-9 | TECHNICAL FIELDS SEARCHED (IPC) |

-/--

The present search report has been drawn up for all claims

| Place of search | Date of completion of the search | Examiner |
|---|---|---|
| Munich | 27 May 2024 | Mühlbauer, Max |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

page 3 of 5

Europäisches
Patentamt

European
Patent Office

Office européen
des brevets

# EUROPEAN SEARCH REPORT

Application Number

EP 23 22 0881

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | TSOMPOU PANAGIOTA I ET AL: "Computational modeling of atherosclerotic plaque progression through an efficient 3D agent-based modeling approach", 2022 IEEE-EMBS INTERNATIONAL CONFERENCE ON BIOMEDICAL AND HEALTH INFORMATICS (BHI), IEEE, 27 September 2022 (2022-09-27), pages 1-4, XP034219338, DOI: 10.1109/BHI56158.2022.9926888 [retrieved on 2022-11-04] * the whole document * | 1-9 | |
| A | MANTZARIS MICHALIS D ET AL: "Computational modeling of atherosclerotic plaque progression in carotid lesions with moderate degree of stenosis*", 2021 43RD ANNUAL INTERNATIONAL CONFERENCE OF THE IEEE ENGINEERING IN MEDICINE & BIOLOGY SOCIETY (EMBC), IEEE, 1 November 2021 (2021-11-01), pages 4209-4212, XP034041578, DOI: 10.1109/EMBC46164.2021.9630376 [retrieved on 2021-11-29] * the whole document * | 1-9 | TECHNICAL FIELDS SEARCHED (IPC) |
| A | KIGKA VASSILIKI I ET AL: "A Machine Learning Model for the Identification of High risk Carotid Atherosclerotic Plaques", 2021 43RD ANNUAL INTERNATIONAL CONFERENCE OF THE IEEE ENGINEERING IN MEDICINE & BIOLOGY SOCIETY (EMBC), IEEE, 1 November 2021 (2021-11-01), pages 2266-2269, XP034043401, DOI: 10.1109/EMBC46164.2021.9630654 [retrieved on 2021-11-29] * the whole document * | 1-9 | |

-/--

The present search report has been drawn up for all claims

| Place of search | Date of completion of the search | Examiner |
|---|---|---|
| Munich | 27 May 2024 | Mühlbauer, Max |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

European Patent Office
Europäisches Patentamt
European Patent Office
Office européen des brevets

## EUROPEAN SEARCH REPORT

**Application Number**

EP 23 22 0881

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | KIGKA VASSILIKI I ET AL: "Early Diagnosis of Carotid Artery Disease based on non-imaging data", 2023 IEEE EMBS SPECIAL TOPIC CONFERENCE ON DATA SCIENCE AND ENGINEERING IN HEALTHCARE, MEDICINE AND BIOLOGY, IEEE, 7 December 2023 (2023-12-07), pages 129-130, XP034533139, DOI: 10.1109/IEEECONF58974.2023.10404873 [retrieved on 2024-01-29] * the whole document * ----- | 1-9 | |
| A | SAVVAS KYRIAKIDIS: "An All-in-One Tool for 2D Atherosclerotic Disease Assessment and 3D Coronary Artery Reconstruction", JOURNAL OF CARDIOVASCULAR DEVELOPMENT AND DISEASE, vol. 10, no. 3, 19 March 2023 (2023-03-19), page 130, XP093166784, ISSN: 2308-3425, DOI: 10.3390/jcdd10030130 * the whole document * ----- | 1-9 | |

TECHNICAL FIELDS SEARCHED (IPC)

The present search report has been drawn up for all claims

| Place of search | Date of completion of the search | Examiner |
|---|---|---|
| Munich | 27 May 2024 | Mühlbauer, Max |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.............................................................................

& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **PASTERKAMP G et al.** *Nat Rev Cardiol.*, January 2017, vol. 14 (1), 21-29
- **VANLAMMEREN GW et al.** *Circulation*, 2014, vol. 129 (22), 2269-76
- **STONE GW et al.** *N Engl J Med.*, 20 January 2011, vol. 364 (3), 226-35
- **BURKE AP et al.** *Circulation*, 2001, vol. 103 (7), 934-40
- **MANN J ; DAVIES MJ**. *Heart*, September 1999, vol. 82 (3), 265-8
- **NAYLOR AR et al.** *Eur J Vasc Endovasc Surg.*, 2018, vol. 55, 3e81
- **ANBEEK P et al.** *Neuroimage*, 2005, vol. 27 (4), 795-804
- **KERKTOLD K et al.** *J. Biomech*, 1991, vol. 24 (6), 409-420
- **VUKICEVIC AM et al.** *Med. Biol. Eng. Comput.*, 2014, vol. 52, 539-556
- **VUKICEVIC AM et al.** *Sci Rep.*, 2018, vol. 8, 1711
- **VUKICEVIC AM et al.** *Sci Rep*, 2018, vol. 8, 1711
- **METZT et al.** *Medical physics.*, 2009, vol. 36, 5568-5579
- **KIGKA VI et al.** *Biomed. Signal Process. Control.*, 2018, vol. 40, 286-294
- **PEZOULAS et al.** *IEEE Open J. Eng. Med. Biol.*, 2020, vol. 1, 83-90
- **PELISEK et al.** *J Clin Med*, 2019, vol. 8 (2)
- **KONTUSH A.** *Curr Opin Lipidol*, 2010, vol. 21 (4), 312-8
- **SAKELLARIOS et al.** *Am. J. Physiol. Heart Circ. Physiol.*, 2013, vol. 304 (11), H1455-1470
- *Annu Int Conf IEEE Eng Med Biol Soc.*, 2020, vol. 2020, 2808-2811
- **STONE et al.** *Circulation*, 2012, vol. 126, 172-181
- **CORTI et al.** *Comput. Biol. Med*, vol. 118, 103623
- **BENTZON et al.** *Circ Res*, vol. 114 (12), 1852-1866
- **SAMADY et al.** *Circulation*, 2011, vol. 124 (7), 779-788
- **GARBEY et al.** *J. Theor. Biol.*, 2017, vol. 429, 149-163
- **KOJIC M et al.** *PAK-F Finite Element Program for Laminar Flow of Incompressible Fluid and Heat Transfer. Kragujevac, Serbia*, 1998
- **FILIPOVIC N et al.** PAK Athero, Finite element program for atherosclerosis. *BIOIRC doo Kragujevac*, 2022
- **TENG Z et al.** *Acta Biomater.*, 2014, vol. 10 (12), 5055-5063